(19) **Europäisches Patentamt**
**European Patent Office**
**Office européen des brevets**

(11) **EP 2 763 620 B1**

(12) **EUROPEAN PATENT SPECIFICATION**

(45) Date of publication and mention
of the grant of the patent:
**30.05.2018 Bulletin 2018/22**

(21) Application number: **12853465.8**

(22) Date of filing: **29.02.2012**

(51) Int Cl.:
*A61B 17/00* (2006.01)        *A61B 34/30* (2016.01)
*A61B 34/20* (2016.01)

(86) International application number:
**PCT/US2012/027121**

(87) International publication number:
**WO 2013/081656 (06.06.2013 Gazette 2013/23)**

(54) **SYSTEM FOR AUTOMATICALLY INITIALIZING OR INITIATING A MOTION COMPENSATION ALGORITHM**

SYSTEM ZUR AUTOMATISCHEN INITIALISIERUNG ODER INITIIERUNG EINES BEWEGUNGSKOMPENSATIONSALGORITHMUS

SYSTÈME D'INITIALISATION OU DE DÉCLENCHEMENT AUTOMATIQUE D'UN ALGORITHME DE COMPENSATION DU MOUVEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2011 US 201113306596**

(43) Date of publication of application:
**13.08.2014 Bulletin 2014/33**

(73) Proprietor: **St. Jude Medical Atrial Fibrillation Division Inc.**
**St. Paul, Minnesota 55117-9913 (US)**

(72) Inventor: **OLSON, Eric S.**
**Maplewood, Minnesota 55119-5838 (US)**

(74) Representative: **Kramer Barske Schmidtchen Patentanwälte PartG mbB**
**European Patent Attorneys**
**Landsberger Strasse 300**
**80687 München (DE)**

(56) References cited:
**WO-A1-00/54689          US-A- 6 144 875**
**US-A1- 2004 254 437     US-A1- 2006 247 621**
**US-A1- 2009 037 130     US-A1- 2010 063 514**
**US-A1- 2010 063 514     US-A1- 2011 160 570**
**US-A1- 2011 164 035**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

a. Field of the Invention

This disclosure relates motion compensation algorithms used to compensate for movement of medical devices disposed within an anatomic structure. This disclosure relates to compensating for movement that is the result of, for example, respiratory activity. The invention relates to a system for automatically initializing or initiating the execution of a motion compensation algorithm.

b. Background Art

Numerous technologies have been developed to permit the determination of the location of a medical device, such as, for example, a catheter, inside of an anatomic structure, such as, for example, the heart. Generally speaking, these technologies allow for the determination of the x, y and z-axis coordinates that define the position of a sensor mounted in or on the medical device, and hence, the position of the medical device.

One common use of the medical device position is for displaying a representation of the medical device with respect to images or models of anatomic structures, such as, for example, images or models of cardiac geometries, within which the medical device is disposed. However, activity such as patient respiration can make the displayed medical device appear to "move" with respect to the images or models of the anatomic structures, which are often created to appear static, e.g., by gating the point in time that the image or model's data is gathered. Similarly, in an instance wherein the medical device is moving, a visualization, navigation, and/or mapping system that may be used to, among other things, generate and cause to be displayed a representation of the medical device, may report motion that includes an artifact component resulting from patient respiration making the overall motion non-representative of the true motion of the medical device. In order to reduce the apparent motion of the medical device with respect to static models or images, or to reduce the artifact component of reported motion, and provide a clinician with a more stable and accurate view, it is known to use motion compensation to correct for the effects introduced by, for example, patient respiratory activity.

In this regard, motion compensation algorithms have been developed that involve determining a respiration motion artifact, which in turn is then subtracted from a calculated (uncompensated) sensor position. While these respiration compensation approaches improve accuracy, they are not without their disadvantages. For example, in at least one conventional approach, the medical device must be stationary (*i.e.*, the medical device is not experiencing commanded movement from a user or automated guidance system) for the motion compensation algorithm to accurately determine a motion compensation signal that is used to generate compensated sensor location data. However, because to this point there has been no means by which to determine and ensure that the medical device is in fact stationary, other than by user involvement, the algorithm or the execution thereof, in particular, has to be initialized or initiated manually by the user. Accordingly, the execution of the motion compensation algorithm may be initialized or later initiated at any time but only at the user's explicit direction.

Accordingly, the inventor hereof has recognized a need for a system for initializing or initiating a motion compensation algorithm that will minimize and/or eliminate one or more of the deficiencies in conventional approaches/systems.

US 2004/0254437 A1 relates to a computer based medical system that can be used to position and navigate electrophysiology catheters, comprising electrodes located on the catheter and swept over a surface of a heart, wherein respiration motion artifacts are measured and subtracted from a position measured by the electrode to improve accuracy

US 2011/0160570 A1 relates to detection methods for use in medical systems, in particular to prolapse and tool/device dislodgement detection systems and methods.

US 2010/0063514 A1 relates to a device and a method for a medical intervention on or in a moving region of a patient body, wherein a medical instrument is introduced into the moving region of the patient's body and guided.

WO 00/54689 relates to an apparatus and method for compensating for respiratory and patient motion during treatment.

US 2009/0037130 A1 relates to a method for recording measurement data from a patient.

BRIEF SUMMARY OF THE INVENTION

The present invention is directed to a system for automatically initializing or initiating a motion compensation algorithm.

In accordance with one aspect of the invention and the present teachings, a system for automatically initializing or initiating a motion compensation algorithm includes a processing apparatus configured to acquire data representing information relating to movement of a medical device, such as, for example, information relating to commanded movement of the medical device. In an exemplary embodiment, the processing apparatus is configured to acquire the data from one of a number of sources, such as, for example, an automated medical device guidance system, a sensor configured to detect movement of the medical device, and a visualization, navigation, and/or mapping system. In an exemplary embodiment wherein the data is acquired from a sensor configured to detect movement of the medical device, the sensor is configured to generate an electrical signal containing the data, and the processing apparatus is configured to receive the signal from the sensor. In an exemplary embodiment, the sensor comprises one of an optical sensor and a magnetic sensor.

The system comprises the medical device itself, namely a catheter and optionally a sheath. The medical device comprises a shaft that may include at least one marker disposed thereon. In such an embodiment, a sensor configured to detect movement of the medical device is further configured to detect movement of the marker and to generate an electrical signal in response thereto.

The processing apparatus is further configured to determine whether the medical device is stationary based on the information represented by the acquired data. In an exemplary embodiment, the processing apparatus is configured to do so by determining whether there has been commanded movement of the medical device within a predetermined period of elapsed time.

The processing apparatus is still further configured to automatically initiate the execution of the motion compensation algorithm in response to a determination that the medical device is stationary.

In an exemplary embodiment, the processing apparatus is further configured to assess predetermined criteria to determine when to initiate the execution of the algorithm, and to initiate the execution when it is determined that the medical device is stationary and certain of the predetermined criteria are met. In an exemplary embodiment, the processing apparatus is configured to initiate the execution of the algorithm when at least one of the following criteria are met: when a distance moved by the medical device since a most recent execution of the algorithm exceeds a predetermined distance threshold; when a predetermined amount of time has elapsed since a most recent execution of the algorithm; when a commanded movement of the medical device results in a distance moved by the medical device within a predetermined period of elapsed time that exceeds a predetermined distance threshold; when the medical device is determined to be disposed within a volumetric region of an anatomic structure for which the algorithm has not been previously executed; and when the processing apparatus determines that a respiration artifact exists in location data corresponding to a location of the medical device, and the artifact has such a magnitude that compensation is required.

In accordance with another aspect of the present disclosure not forming part of the invention, a method of automatically initializing or initiating a motion compensation algorithm comprises acquiring data representing information relating to movement of a medical device. The acquiring step comprises acquiring the data from one of: an automated medical device guidance system; a sensor configured to detect movement of the medical device; and a visualization, navigation, and/or mapping system.

In an example the acquiring step comprises acquiring the data from a sensor, the acquiring step comprises receiving an electrical signal from a sensor configured to detect movement of the medical device, wherein the electrical signal contains the data representing information relating to commanded movement of the medical device.

The method further comprises determining whether the medical device is stationary based on the information represented by the acquired data. The method still further comprises automatically initiating the execution of the algorithm in response to a determination that the medical device is stationary.

In an example, the method further comprises assessing predetermined criteria to determine when to initiation the execution of the algorithm. In an example, the initiating step comprises automatically initiating the execution of the algorithm when the medical device is determined to be stationary and certain of the predetermined criteria are met. In an example, the initiating step comprises automatically initiating the execution of the algorithm when at least one of the following criteria are met: when a distance moved by the medical device since a most recent execution of the algorithm exceeds a predetermined distance threshold; when a predetermined amount of time has elapsed since a most recent execution of the algorithm; when a commanded movement of the medical device results in a distance moved by the medical device within a predetermined period of elapsed time that exceeds a predetermined distance threshold; when the medical device is determined to be disposed within a volumetric region of an anatomic structure for which the algorithm has not been previously executed; and when the processing apparatus determines that a respiration artifact exists in location data corresponding to a location of the medical device, and the artifact has such a magnitude that compensation is required.

The foregoing and other aspects, features, details, utilities, and advantages of the present invention will be apparent from reading the following description and claims, and from reviewing the accompanying drawings.

BRIEF DESCRIPTION OF THE DRAWINGS

Fig. 1 is a diagrammatic view of a system for automatically initializing or initiating the execution of a motion compensation algorithm in accordance the present teachings.

Fig. 2 is a simplified diagrammatic and schematic view of an exemplary visualization, navigation, and/or mapping system of the system illustrated in Fig. 1.

Figs. 3A-3D are schematic diagrams of exemplary dipole pairs of driven patch electrodes suitable for use in a visualization, navigation, and/or mapping system of the system illustrated in Fig. 1.

Fig. 4 is a flow chart illustrating methods for automatically initializing or initiating the execution of a motion compensation algorithm.

Fig. 5A is an isometric view of an exemplary embodiment of an arrangement of a catheter and an introducer of the system illustrated in Fig. 1.

Fig. 5B is an enlarged view of the portion of the catheter and introducer arrangement identified as "5B" in Fig. 5A.

Fig. 6 is an isometric diagrammatic view of a robotic-based automated medical device guidance system illustrating an exemplary layout of various system components.

Fig. 7 is an elevation view of an exemplary medical device manipulator support structure of the robotic-based system illustrated in Fig. 6, including a medical device manipulator assembly.

Fig. 8 is a diagrammatic view of the robotic-based system illustrated in Fig. 6 including an isometric view of a device cartridge of the manipulator assembly illustrated in Fig. 7.

Fig. 9 is a diagrammatic view of a magnetic-based automated medical device guidance system.

DETAILED DESCRIPTION OF THE INVENTION

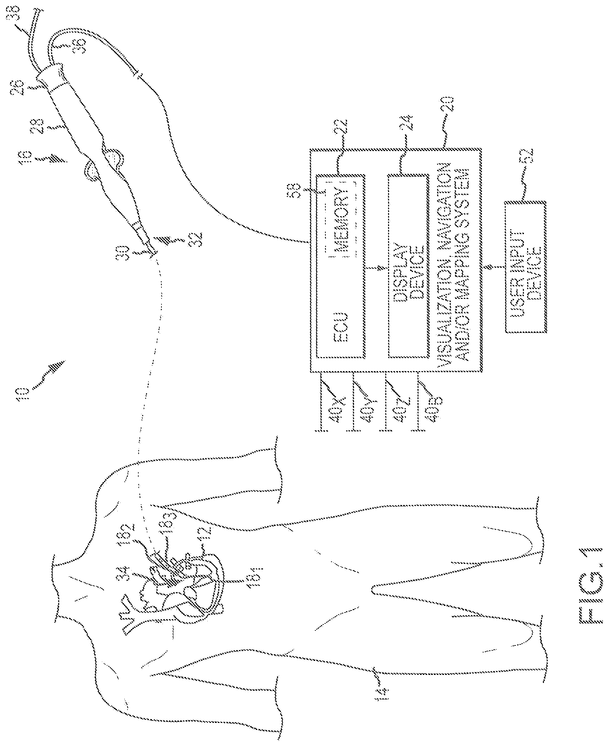

Referring now to the drawings wherein like reference numerals are used to identify identical components in the various views, Fig. 1 illustrates one exemplary embodiment of a system 10 for performing one more diagnostic and/or therapeutic functions. More particularly, the system 10 includes components for, among other things, generating surface models of anatomic structures, such as, for example and without limitation, a heart 12 of a body 14. It should be understood, however, that while the description below is with respect to a heart, the system 10 may find application in connection with a variety of other anatomic structures within human and non-human bodies.

Among other components, the system 10 includes one or more medical devices 16, such as, for example, a catheter, a sheath, an introducer, and the like. The present invention may be used with a catheter, a sheath, an introducer, a stent, a guide wire, or another medical device. For purposes of clarity and illustration, however, the description below will be with respect to the medical device 16 comprising a catheter (catheter 16). As will be described in greater detail below, the catheter 16 has one or more sensors 18 mounted therein or thereon. The system 10 may further include a system 20 for the visualization, navigation, and/or mapping of internal body structures, which may include a processing apparatus, such as an electronic control unit (ECU) 22, and a display device 24. Alternatively, one or both of the ECU 22 and the display 24 may be separate and distinct from, but electrically connected to and configured for communication with, the system 20.

With continued reference to Fig. 1, the catheter 16 is provided for examination, diagnosis, and/or treatment of internal anatomic structures, such as, for example, the heart 12, and the tissue thereof, in particular. In an exemplary embodiment, the catheter 16 comprises an ablation catheter, such as an irrigated radio-frequency (RF) ablation catheter. It should be understood, however, that catheter 16 is not limited to an irrigated and/or RF-based ablation catheter, or to an ablation catheter at all. Rather, in other exemplary embodiments, the catheter 16 may comprise a non-irrigated catheter and/or other types of ablation catheters (e.g., cryoablation, ultrasound, etc.), or other therapeutic and/or diagnostic catheters.

The catheter 16 may include a cable connector or interface 26, a handle 28, a shaft 30 having a proximal end 32 and a distal end 34 (as used herein, "proximal" refers to a direction toward the end of the catheter 16 near the clinician, and "distal" refers to a direction away from the clinician and (generally) inside the body of a patient), and one or more sensors, such as sensors 18 (*i.e.*, $18_1$, $18_2$,...,$18_N$), mounted in or on the shaft 30 of the catheter 16. In an exemplary embodiment, the sensors 18 are disposed at or near the distal end 34 of the shaft 30. The catheter 16 may further include a combination of other components such as, for example and without limitation, a temperature sensor, additional electrodes, ablation elements (e.g., ablation tip electrodes for delivering RF ablative energy, high intensity focused ultrasound ablation elements, etc.), and corresponding conductors or leads.

The connector 26 provides mechanical, fluid, and electrical connection(s) for cables, such as, for example, cables 36, 38 extending to and from the visualization, navigation, and/or mapping system 20, and possibly other components of the system 10 (*e.g.*, an ablation generator, irrigation source, etc.). The connector 26 is conventional in the art and is disposed at the proximal end 32 of the catheter 16.

The handle 28 provides a location for the clinician to hold the catheter 16 and may further provide means for steering or guiding the shaft 30 within the body 14. For example, the handle 28 may include means to change the length of a steering wire extending through the catheter 16 to the distal end 34 of the shaft 30 to steer the shaft 30. The handle 28 is also conventional in the art and it will be understood that the construction of the handle 28 may vary. In another exemplary embodiment, and as will be described in greater detail below, the catheter 16 may be driven or controlled by an automated medical device guidance system, such as, for example and without limitation, a robotic or a magnetic-based system. Accordingly, in such an embodiment, rather than a clinician manipulating a handle to steer or guide the catheter 16, and the shaft 30 thereof, in particular, an automated medical device guidance system is configured to manipulate the catheter 16. One exemplary robotic system is described and depicted in U.S. Patent Publication No. 2009/0247993 entitled "Robotic Catheter System,".

The shaft 30 is an elongate, tubular, flexible member configured for movement within the body 14. The shaft 30 supports, for example and without limitation, sensors mounted therein or thereon, such as, for example, the sensors 18, associated conductors, and possibly additional electronics used for signal processing or conditioning. The shaft 30 may also permit transport, delivery and/or removal of fluids (including irrigation fluids, cryogenic ablation fluids, and bodily fluids), medicines, and/or surgical tools or instruments. The shaft 30 may be made from conventional materials such as polyurethane, and defines one or more lumens configured to house and/or transport electrical conductors, fluids, or surgical tools. The shaft 30 may be introduced into a blood vessel or other structure within the body 14 through a conventional introducer. The shaft 30 may then be steered or guided through the body 14 to a desired location using means well known in the art.

The sensors 18 mounted in or on the shaft 30 of the catheter 16 may be provided for a variety of diagnostic and therapeutic purposes including, for example, electrophysiological studies and cardiac mapping. In embodiments of the invention, one or more of the sensors 18 are provided to perform a position sensing function. More particularly, and as will be described in greater detail below, one or more of the sensors 18 are configured to be positioning sensors that provide information to, for example, the visualization, navigation, and mapping system 20 relating to the location (*e.g.*, position and orientation) of the catheter 16, and the distal end 34 thereof, in particular, at certain points in time. Accordingly, in the illustrated embodiment, the catheter 16 includes a plurality of sensors 18, such as sensors $18_1$, $18_2$, $18_3$, disposed in or on the shaft 30 at or near the distal end 34 thereof, some or all of which are electrically connected to the visualization, navigation, and mapping system 20 and/or other components of the system 10. As will be described below, the sensors 18 may comprise one of a number of types of sensors, such as, for example and without limitation, electrodes (*e.g.*, tip electrodes and ring electrodes) or magnetic sensors (*e.g.*, magnetic coils). It will be appreciated that while only certain embodiments of the catheter 16 having particular numbers and types of sensors mounted therein or thereon are described in detail herein, the number, shape, orientation, and purpose of the sensors may vary, and embodiments wherein the catheter 16 has sensors different from those specifically described herein remain within the spirit and scope of the present disclosure.

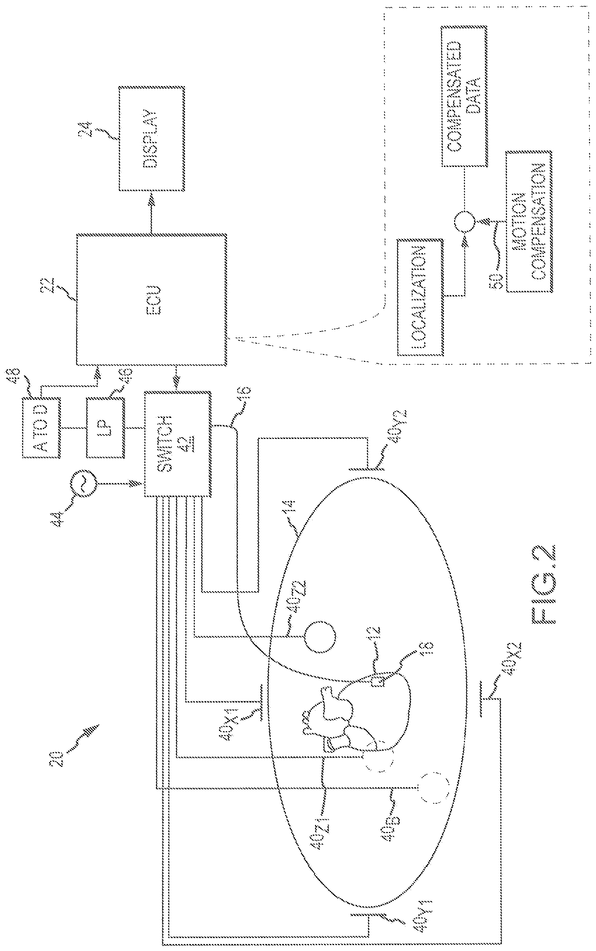

With reference to Figs. 1 and 2, the visualization, navigation, and mapping system 20 will be described. The system 20 is provided for visualization, navigation, and/or mapping of internal body structures. The visualization, navigation, and/or mapping system 20 may comprise an electric field-based system, such as, for example, that having the model name EnSite NavX™ and commercially available from St. Jude Medical, Inc., and as generally shown with reference to U.S. Patent No. 7,263,397 entitled "Method and Apparatus for Catheter Navigation and Location and Mapping in the Heart,". In other exemplary embodiments, however, the visualization, navigation, and/or mapping system may comprise other types of systems, such as, for example and without limitation: a magnetic-field based system such as the Carto™ System available from Biosense Webster, and as generally shown with reference to one or more of U.S. Patent Nos. 6,498,944 entitled "Intrabody Measurement," 6,788,967 entitled "Medical Diagnosis, Treatment and Imaging Systems," and 6,690,963 entitled "System and Method for Determining the Location and Orientation of an Invasive Medical Instrument," or the gMPS system from MediGuide Ltd., and as generally shown with reference to one or more of U.S. Patent Nos. 6,233,476 entitled "Medical Positioning System," 7,197,354 entitled "System for Determining the Position and Orientation of a Catheter," and 7,386,339 entitled "Medical Imaging and Navigation System,"; a combination electric field-based and magnetic field-based system such as the Carto 3™ System also available from Biosense Webster; as well as other impedance-based localization systems, acoustic or ultrasound-based systems, and commonly available fluoroscopic, computed tomography (CT), and magnetic resonance imaging (MRI)-based systems.

In an exemplary embodiment, and as briefly described above, the catheter 16 includes one or more positioning sensors 18 for producing signals indicative of catheter position and/or orientation information. As will be described in greater detail below, the position and orientation of the catheter 16, and the sensors 18 thereof, in particular, may be used in the generation of, for example, surface models of anatomic structures, as well as various types of maps, such as, for example, electrophysiological (EP) maps, tissue morphology maps, and the like. In an embodiment wherein the system 20 is an electric field-based system, the positioning sensor(s) 18 may include one or more electrodes mounted in or on the shaft 30 of the catheter 16.

Alternatively, in an embodiment wherein the system 20 is a magnetic field-based system, the positioning sensor(s) 18 may comprise one or more magnetic sensors configured to detect one or more characteristics of a low-strength magnetic field. For instance, in one exemplary embodiment, the magnetic sensors may comprise magnetic coils.

For purposes of clarity and illustration, the system 20 will hereinafter be described as comprising an electric field-based system, such as, for example, the EnSite NavX™ system identified above. Accordingly, it will be appreciated that while the description below is primarily limited to an embodiment wherein the positioning sensor(s) 18 comprises one or more positioning electrodes, in other exemplary embodiments, the positioning sensors 18 may comprise one or more magnetic field sensors (e.g., coils) or dedicated positioning electrodes that are separate and distinct from the sensors 18. Accordingly, visualization, navigation, and mapping systems that include positioning sensors other than the electrodes 18 described below, or electrodes in general, remain within the scope of the present disclosure.

With continued reference to Figs. 1 and 2, the system 20 may include a plurality of patch electrodes 40, the ECU 22, and the display device 24, among other components. However, as briefly described above, in another exemplary embodiment, the ECU 22 and/or the display device 24 may be separate and distinct components that are electrically connected to, and configured for communication with, the system 20.

With the exception of the patch electrode $40_B$ called a "belly patch," the patch electrodes 40 are provided to generate electrical signals used, for example, in determining the position and orientation of the catheter 16, and in the guidance thereof. In one embodiment, the patch electrodes 40 are placed orthogonally on the surface of the body 14 and are used to create axes-specific electric fields within the body 14. For instance, in one exemplary embodiment, patch electrodes $40_{X1}$, $40_{X2}$ may be placed along a first (x) axis. Patch electrodes $40_{Y1}$, $40_{Y2}$ may be placed along a second (y) axis, and patch electrodes $40_{Z1}$, $40_{Z2}$ may be placed along a third (z) axis. In other embodiments, the dipoles created may not be on an axis, for example, a dipole between electrodes $40_{X1}$ and $40_{Y1}$. Each of the patch electrodes 40 may be coupled to a multiplex switch 42. In an exemplary embodiment, the ECU 22 is configured through appropriate software to provide control signals to switch 42 to thereby sequentially couple pairs of electrodes 40 to a signal generator 44. Excitation of each pair of electrodes 40 generates an electrical field within body 14 and within an area of interest such as the heart 12. Voltage levels at non-excited electrodes 40, which are referenced to the belly patch $40_B$, are filtered by, for example, a low pass filter 46, converted by an analog-to-digital converter 48, and provided to ECU 22 for use as reference values.

As briefly discussed above, in an exemplary embodiment, the catheter 16 includes one or more electrodes 18 mounted therein or thereon that are electrically connected to the ECU 22 and that is/are configured to serve a position sensing function. In an exemplary embodiment, the electrodes 18 are placed within electrical fields created in the body 14 (e.g., within the heart 12) by exciting the patch electrodes 40. For purposes of clarity and illustration, the description below will be limited to an embodiment wherein a single electrode 18 is placed within the electric fields. It will be appreciated, however, that in other exemplary embodiments that remain within the spirit and scope of the present disclosure, a plurality of electrodes 18 can be placed within the electric fields and then positions and orientations of each electrode can be determined using the techniques described below.

When disposed within the electric fields, the electrode 18 experiences voltages that are dependent on the location between the patch electrodes 40 and the position of the electrode 18 relative to the tissue of the anatomic structure. Voltage measurement comparisons made between the electrode 18 and the patch electrodes 40 can be used to determine the position of the electrode 18 relative to the anatomic structure. This position information may then be used by the ECU 22, for example, to generate models, such as surface models, and/or maps of, or corresponding to, the anatomic structure, as is well known in the art. Accordingly, as the catheter 16 is moved about along the surface of a desired anatomic structure, for example, the electrode 18 can be used to collect location data points that correspond to locations of the electrode 18, and therefore, the surface of the anatomic structure. These location data points can then be used by the ECU 22, for example, to generate or construct a surface model of the anatomic structure. Further, information received from the electrode 18 can also be used to display on a display device, such as display device 24, the location and orientation of the electrode 18 and/or the tip of the catheter 16. Accordingly, among other things, the ECU 22 of the system 20 provides a means for generating display signals used to the control display device 24 and the creation of a graphical user interface (GUI) on the display device 24.

In addition to the above, the ECU 22 may further provide a means for controlling various components of system 10 including, but not limited to, the switch 42. It should be noted that while in an exemplary embodiment the ECU 22 is configured to perform some or all of the functionality described above and below, in another exemplary embodiment, the ECU 22 may be separate and distinct from the system 20, and system 20 may have another processor configured to perform some or all of the functionality described herein (e.g., acquiring the position/location of the electrode/catheter, for example). In such an embodiment, the processor of the system 20 would be electrically coupled to, and configured for communication with, the ECU 22. For purposes of clarity and ease of description, however, the description below will be limited to an embodiment wherein ECU 22 is part of system 20 and configured to perform all of the functionality described herein.

The ECU 22 may comprise a programmable microprocessor or microcontroller, or may comprise an application specific integrated circuit (ASIC). The ECU 22 may include a central processing unit (CPU) and an input/output (I/O) interface through which the ECU 22 may receive a plurality of input signals including, for example, signals generated by patch electrodes 40 and the electrode 18, and generate a plurality of output signals including, for example, those used to control and/or provide data to treatment devices, the display device 24 and the switch 42. The ECU 22 may be configured to perform various functions, such as those described in greater detail below, with appropriate programming instructions or code (*i.e.*, software). Accordingly, the ECU 22 is programmed with one or more computer programs encoded on a computer storage medium for performing the functionality described herein.

In operation, the ECU 22 generates signals to control the switch 42 to thereby selectively energize the patch electrodes 40. The ECU 22 receives raw position signals (location information or location data representative of location information) from the catheter 16 (and particularly the electrode 18) reflecting changes in voltage levels on the electrode 18 and from the non-energized patch electrodes 40. The ECU 22 uses the raw location data produced by the patch electrodes 40 and electrode 18 to determine the raw, uncompensated electrode location coordinates of the electrode 18 in three-dimensional space (x, y, z).

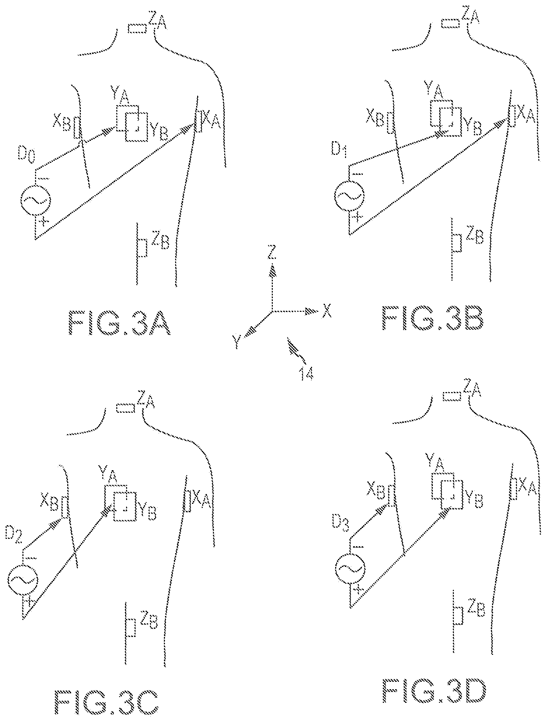

While the description above has thus far been generally with respect to an orthogonal arrangement of the patch electrodes 40, the present disclosure is not meant to be so limited. Rather, in other exemplary embodiments, non-orthogonal arrangements may be used to determine the raw, uncompensated electrode location coordinates of the electrode 18. For example, and in general terms, Figs. 3A-3D depict a plurality of exemplary non-orthogonal dipoles $D_0$, $D_1$, $D_2$, and $D_3$, set in a coordinate system 49. In Figs. 3A-3D, the X-axis patch electrodes are designated $X_A$ and $X_B$, the Y-axis patch electrodes are designated $Y_A$ and $Y_B$, and the Z-axis patch electrodes are designated $Z_A$ and $Z_B$. For any desired axis, the potentials measured across an intra-cardiac sensor, such as electrode 18, resulting from a predetermined set of drive (source sink) configurations may be combined algebraically to yield the same effective potential as would be obtained simply by driving a uniform current along the orthogonal axes. Any two of the patch electrodes $40_{X1}$, $40_{X2}$, $40_{Y1}$, $40_{Y2}$, $40_{Z1}$, and $40_{Z2}$ (See Fig. 2) may be selected as a dipole source and drain with respect to a ground reference, e.g., the belly patch $40_B$, while the unexcited patch electrodes measure voltage with respect to the ground reference. The electrode 18 placed in the heart 12 is also exposed to the field for a current pulse and is measured with respect to ground, *e.g.*, the belly patch $40_B$.

Data sets from each of the patch electrodes and the electrode 18 are all used to determine the location of the electrode 18 within the heart 12. After the voltage measurements are made, a different pair of patch electrodes is excited by the current source and the voltage measurement process of the remaining patch electrodes and internal electrode takes place.

Whether an orthogonal or non-orthogonal arrangement is used to determine the raw, uncompensated electrode location coordinates of the electrode 18 in three-dimensional space, the ECU 22 is further configured, as will be described below, to correct the raw data to account for respiration, cardiac activity, and other artifacts. More particularly, the ECU 22 is configured to generate a compensation signal, designated as signal 50 in Fig. 2, suitable for minimizing or eliminating motion artifacts (*e.g.*, respiration, cardiac, etc.) contained in the determined electrode location. This may be done using known or hereafter developed techniques, including, for example and without limitation, those described below.

As was briefly described above, respiratory activity may make medical devices, such as, for example, catheters, appear to move with respect to models of anatomic structures that appear static in nature. In order to reduce the apparent motion of the medical devices, a compensation function is used, as produced by, for example, the ECU 22. The particular compensation function used - or the motion compensation algorithm (referred to herein as "motion compensation algorithm" or "algorithm") configured to generate the compensation function - is dependent, of course, on the type of visualization, navigation and mapping system being employed (*e.g.*, electric or magnetic field-based systems). For purposes of consistency, clarity, and illustration, the description below will be limited to an embodiment wherein the visualization, navigation, and mapping system 20 is an electric field-based system, and thus the exemplary motion compensation algorithm described below will be one for use with an electric field-based visualization, navigation, and mapping system. It will be appreciated, however, that in other exemplary embodiments, visualization, navigation, and mapping systems other than electric field-based systems may be used, and compensation algorithms specifically tailored to such systems would be employed, the execution of which could be initiated in the same manner described in greater detail below.

One exemplary compensation algorithm that may be used is that described in U.S. Patent No. 7,263,397 entitled "Method and Apparatus for Catheter Navigation and Location and Mapping in the Heart." To summarize in general terms, however, an exemplary motion compensation algorithm comprises generating a compensation signal 50 (depicted in Fig. 2) corresponding to, and representative of, the undesired respiration artifact, and subtracting the compensation signal 50 from the uncompensated raw location of the electrode 18 so that the true location of the catheter 16 can be determined. In an exemplary embodiment, the ECU 22 is configured to initialize/initiate and execute the motion compensation algorithm described above to generate the compensated sample of data from the electrode 18. However, in other exemplary embodiments that remain within the spirit and scope of the present disclosure, another component of the system 10, such as, for example, another processor or ECU, electrically connected to and configured for communication with the ECU 22 may be used.

In the exemplary motion compensation algorithm, the data from the patches 40 are used to reduce the influence of respiratory effects on location data of the electrode 18. More particularly, when the algorithm is first initialized (*e.g.*, at the beginning of an EP study, for example) or the execution of the algorithm is initiated (*e.g.*, subsequent to the first initialization of the algorithm and later in the course of the EP study, for example), data is acquired from the electrode 18 and a subset of the un-driven patch electrodes 40. In an exemplary variant not forming part of the claimed invention, this phase begins when the user instructs the ECU 22 to execute the algorithm. The user may provide such instructions using a user interface 52 (shown in Fig. 1), such as, for example and without limitation, a touch screen, a keyboard, a keypad, a slider control, a button, or some other user-controllable input device that is electrically connected to the ECU 22 to allow the user to provide instructions to the ECU 22. In other exemplary embodiments, and as will be described in greater detail below, the algorithm may be initialized, or the execution thereof initiated, in a manner other than by the user providing instructions to the ECU 22.

In any event, during the initialization or initiation phase of the algorithm, it is assumed by the ECU 22 that the electrode 18 is stationary or in an anatomically stable position, and that any movement or motion of the electrode 18 observed during the initialization or initiation phase is the result of, for example, respiratory activity. Accordingly, for the purposes of this disclosure, "stationary" or "stable" are intended to mean that the catheter 16, and therefore the electrode 18, is not moving as a result of movement commanded by either a user or, as will be described in greater detail below, an automated medical device guidance system. Such movement will hereafter be referred to as "commanded movement" and is differentiated from movement of the catheter 16 that is not directed or commanded by a user or automated guidance system (*i.e.*, non-commanded movement), such as, for example and without limitation, movement resulting from respiratory activity.

During the initialization or initiation phase, parameters of the algorithm must be set or updated to allow the algorithm to optimally perform. For an exemplary embodiment of the algorithm, assume that "N" samples of raw location data corresponding to various locations of the electrode 18 are acquired. Note that in an embodiment wherein the catheter 16 has a plurality of electrodes 18, location data samples may be acquired for each positioning electrode 18 and processed in the manner described below. Each sample of location data for the electrode 18, which comprises x, y, z-axis data, is denoted as "$E_i$", where "i" ranges from 1 to N. In addition to location data corresponding to the electrode 18, data from a subset of the patch electrodes 40 is also acquired. More particularly, data from "P" un-driven patch electrodes 40 is acquired, and is denoted as "$R_{ki}$", where "k" ranges from 1 to P and "i" ranges from 1 to N. For purposes of clarity and illustration, the description below will only be with respect to the x-axis component of the electrode data. It will be appreciated, however, that the invention described herein may be used on any axis, e.g., it may also be employed on the y- or the z-axis, and if so these components are treated identically and independently in the same manner as that described below. Likewise, the invention may be employed on multiple axes, such as the x- and y- axes, or on all three.

Since the electrode 18 is assumed to be stable when the algorithm is initialized or initiated, the mean of the electrode data is subtracted out from the electrode data $E_i$ to obtain a mean-subtracted "noise" signal, which is denoted as "$E_{msi}$", and is calculated using equation (1):

$$(1) \qquad E_{msi} = E_i - \frac{1}{N} \sum_{i=1}^{N} E_i$$

Ideally, the calculated noise would be as close to zero as possible, and properly weighted data from the patch electrodes 40 can be used to drive to that goal. In order to find the most appropriate weights, equation (2) below is used:

$$(2) \qquad \sum_{i=1}^{N} \left[ E_{msi} - (\sum_{k=1}^{P} R_{ki} * W_k) \right]^2$$

This is a linear least squares problem and it is amenable to singular value decomposition or other linear solution methods. In other words, in the linear system solution, a set of P weights (W) are determined, such that when each weight is multiplied by its respective R vector, as defined above, and the results are summed, the inner sum is optimally close to the noise (respiration artifact) as measured during the initialization/initiation phase.

Once determined, the weighted sum of the R vectors derived from data from the patch electrodes 40 is subtracted from the uncompensated sample of data from the electrode 18, $E_i$, using the equation (3) below, yielding a net compensated sample "$E_{Ri}$":

$$(3) \qquad E_{Ri} = E_i - (\sum_{k=1}^{P} R_{ki} * W_k).$$

It should be noted that each time the algorithm is initialized or initiated (run) following the first initialization, the parameters of the algorithm (*e.g.*, $E_i$, $R_{ki}$, $W_k$) must be updated or reset to ensure the algorithm operates properly and optimally. In other words, in an exemplary embodiment, no information is retained from any previous execution of the compensation algorithm, and thus, each time the algorithm is run or executed, the process described above is repeated. In another embodiment, while the information is retained from a previous execution, it is only a starting point for the system to interpolate to the current parameters. Once the parameters of the algorithm are set, the execution of the algorithm may proceed to the calculation phase described above with respect to equation (3) during which the net compensation sample $E_{Ri}$ is calculated.

In an exemplary embodiment, some or all of the data used in the calculations described above (*e.g.*, location data for the electrode 18, data for one or more subsets of the patch electrodes 40, etc.) may be collected in a rolling buffer such that once a determination is made that the catheter 16 is stationary, the compensation algorithm can be immediately executed. One purpose for this is that even if the catheter 16 moves immediately after it has been determined to be stationary, the system will not be prevented from making the necessary computations since the data is stored in the rolling buffer.

It will be appreciated by those of ordinary skill in the art that while the compensation algorithm described above is the only compensation algorithm described in detail, the present disclosure is not meant to be limited to only that particular above-described algorithm. Rather, other compensation algorithms, such as, for example and without limitation, linear solutions other than that described above and non-linear solutions, may be used and remain within the spirit and scope of the present disclosure.

As briefly described above, provided the catheter 16 and the electrode 18 thereof, in particular, is considered to be stationary or stable (which is a condition that must be met to execute the algorithm) the algorithm may be first initialized and thereafter the execution initiated in number of ways.

One known way this may be accomplished is by manually initializing or initiating the algorithm. More particularly, a user of the system may manually initialize or initiate the execution of the algorithm by instructing the ECU 22 to do so when the user determines that the catheter 16 is stationary (*e.g.*, because the user knows when movement of the catheter 16 is not commanded, the user knows there is no commanded movement and/or can visually tell that the catheter 16 is not moving simply by looking at a monitor that contains on image of the catheter 16, for example). More particularly, the user may instruct the ECU 22 to initialize or initiate the execution of the algorithm via a user interface, such as, for example, the user interface 52 described above and shown in Fig. 1.

According to the present invention, initializing or initiating the execution of the algorithm is by the ECU 22, automatically initializing or initiating the algorithm. Automatic initialization or initiation may be employed in systems wherein the catheter 16 is manually guided or manipulated by a user (*e.g.*, a physician or clinician), or in automated medical device guidance systems, such as, for example, robotic medical device guidance systems or magnetic-based medical device guidance systems. In the case of automated medical device guidance systems, the system has inherent knowledge at all times as to whether or the catheter 16 that the system controls is moving by commanded movement or is stationary. As will be described in greater detail below, this knowledge allows for the compensation algorithm to be initialized/initiated without any input from a user of the system or, in certain embodiments, from additional sensors. In the case of manually-guided systems, the system could be adapted to record movement of the catheter 16 by use of visual, fluoroscopic, or sensor-based markers to determine when the catheter 16 is stable.

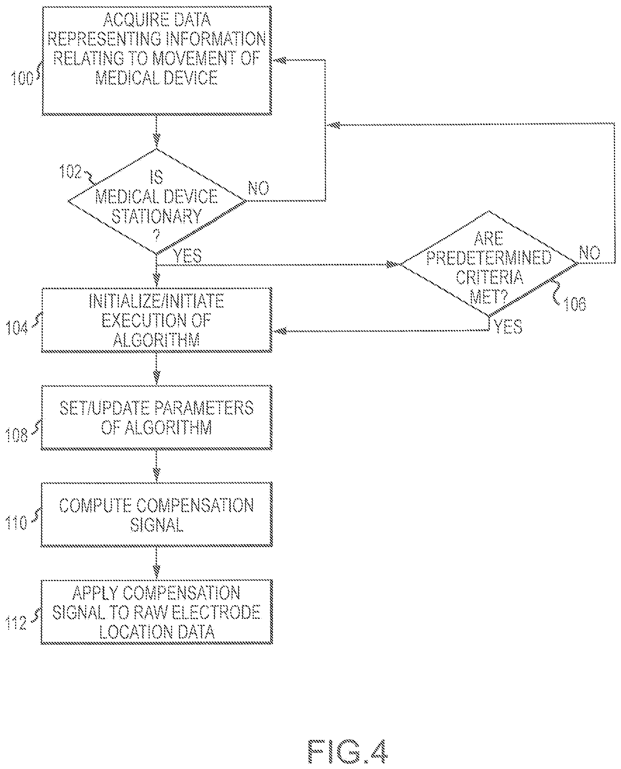

However, in either type of system, and as illustrated in Fig. 4 and described in greater detail below, the ECU 22 is generally configured to acquire data representing information relating to movement of the catheter 16, such as, for example and without limitation, commanded movement (step 100), to determine whether the catheter 16 is stationary based on the information represented by the acquired data (step 102), and to automatically initialize or initiate the execution of the algorithm in response to a determination that the medical device is stationary (step 104).

With reference to Figs. 4-5B, the automatic initialization or initiation of the algorithm for a user-controlled system for guiding or manipulating a medical device, such as the catheter 16, will be described. In such an embodiment, and as briefly described above, the ECU 22 may be configured to acquire data representing information relating to movement of the catheter 16. The ECU 22 may acquire this data from a number of sources and/or in a number of different ways.

For example, in one embodiment, the ECU 22 is configured to receive one or more electrical signal(s) containing the data. The electrical signal(s) may be received from one or more of a number of sources. In an exemplary embodiment, the catheter 16 includes one or more sensors 54 mounted therein or thereon that are electrically connected to, and configured for communication with, the ECU 22. The sensors 54 are configured to detect movement of the catheter 16 and to generate the electrical signal(s) containing the data. In such an embodiment, the information represented by the data contained in the electrical signal(s) relates to commanded movement of the catheter 16, as opposed to movement caused by, for example, respiratory activity.

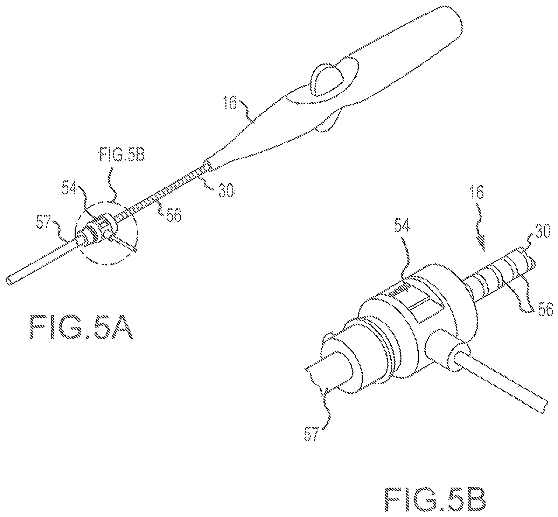

With reference to Figs. 5A and 5B, in another exemplary embodiment, the sensor 54 associated with the catheter 16 is a sensor configured to detect or monitor the movement of one or more markers on the catheter 16. More particularly, in an exemplary embodiment the shaft 30 of the catheter 16 comprises a plurality of markers 56, such as, for example and without limitation, optical grating disposed thereon (thus, the markers may be formed of, for example, materials configured to be optically detected) that can be used in conjunction with an optical sensor. In another exemplary embodiment, rather than optical grating, the markers 56 may comprise magnetic material, such as ferrous material embedded in the shaft 30, or a magnetic strip affixed to or disposed within the shaft 30, that can be used in conjunction with a magnetic sensor. In one exemplary embodiment such as that illustrated in Figs. 5A and 5B, the markers 56 comprise optical grating and are arranged in a pattern where the markers are spaced apart from each other by a distance of one millimeter (1 mm) so as to provide adequate resolution. It will be appreciated, however, that the distance between adjacent markers 56 may be greater than or less than 1 mm.

The sensor 54 may be disposed in or on the catheter 16, or another medical device used in conjunction with the catheter 16 (*e.g.*, the marker(s) 56 may be disposed on the catheter 16, while the sensor 54 may be disposed in or on another medical device (*e.g.*, sheath or introducer) used with, and located in close proximity to, the catheter 16). For example, in the embodiment illustrated in Figs. 5A and 5B, the sensor 54 is disposed in or on an introducer 57 and is configured to read or sense the markers 56 on the shaft 30 of the catheter 16 as the catheter shaft 30 is advanced through the introducer 57. In any event, in the illustrated embodiment the sensor 54 functions as an encoder detecting, monitoring, or tracking the movement of the markers 56, and therefore, the catheter 16.

Accordingly, in an exemplary embodiment, the sensor 54 generates electrical signal(s) that contain data representing information relating to the commanded movement of the catheter 16. The electrical signal(s) are received by the ECU 22, which is electrically connected to the sensor 54. The sensor 54 may comprise any number of sensors known in the art, such as, for example and without limitation, the NSE-5310 miniature position sensor available from New Scale Technologies of Victor, New York, or the HEDR Reflective Optical Encoder Modules available from US Digital of Vancouver, Washington. While these particular sensors are identified with specificity, it will be appreciated that any number of optical, linear, magnetic, resistive, and/or other known types of sensors (encoders) that are well known in the art may be used, and therefore, remain within the spirit and scope of the present disclosure.

In yet another embodiment, a separate rail system may be employed wherein the sensor 54 may comprise a linear strip encoder that is attached to the catheter 16 or another medical device used in conjunction with the catheter 16. More particularly, the encoder (*i.e.*, the sensor 54) may be disposed at or near the distal end 34 of the shaft 30 of the catheter 16 and electrically connected to, and configured for communication with, the ECU 22. Accordingly, the sensor 54 generates the electrical signal(s) containing the data representing information relating to the commanded movement of the catheter 16. In an embodiment wherein the sensor 54 comprises an encoder, the encoder may take any number of forms, such as, for example, optical, linear, magnetic, or resistive, all of which are well known in the art and remain within the spirit and scope of the present disclosure.

In yet still another embodiment, the sensor 54 comprises a linear encoder mounted at or near the handle 28 of the catheter 16 and electrically connected to and configured for communication with the ECU 22. In such an embodiment, the sensor 54 is configured to generate the electrical signal(s) containing the data representing information relating to the commanded movement of the catheter 16.

Regardless of the type of sensor 54, in an embodiment wherein one or more electrical signal(s) are generated by a sensor 54 that is electrically connected to the ECU 22, the electrical signal(s) is received and processed by the ECU 22. The ECU 22 is programmed or configured to process the received electrical signal(s) and to determine whether the catheter 16 is stationary. Accordingly, the ECU 22 is configured to extract the information represented by the data contained in the received electrical signal(s) and to determine, based on the information, whether the catheter 16 is stationary.

In one embodiment, the ECU 22 is configured to make such a determination based solely on the information represented by the data contained in the received electrical signal(s). Accordingly, in such an embodiment, if the information indicates that there is currently commanded movement of the catheter 16, the ECU 22 will determine that the catheter 16 is not stationary, and therefore, as illustrated in Fig. 4, will not initialize or initiate the execution of the algorithm. If, on the other hand, the information indicates that the catheter 16 is not currently experiencing commanded movement, the ECU 22 will determine that the catheter 16 is stationary, and therefore, as illustrated in Fig. 4, will initialize or initiate the execution of the algorithm, provided no other requirements or criteria, such as those described in greater detail below, must be met prior to initialization/initiation.

In another exemplary embodiment, the ECU 22 is configured to make the determination based on the information represented by the data contained in the received electrical signal(s), as well as one or more predetermined conditions. These predetermined conditions may include, for example, that there has been no commanded movement within a predetermined period of elapsed time. For instance, the ECU 22 may be programmed such that it will only determine that the catheter 16 is stationary if there has not been commanded movement of the catheter 16 over the past ten (10) seconds. Accordingly, in such an embodiment, if the information indicates that there has been commanded movement of the catheter 16 within a predetermined period of elapsed time, the ECU 22 will determine that the catheter 16 is not stationary, and therefore, as illustrated in Fig. 4, will not initialize or initiate the execution of the algorithm. On the other hand, if the information indicates that there has not been commanded movement of the catheter 16 within the predetermined period of elapsed time, the ECU 22 will determine that the catheter 16 is stationary, and therefore, as illustrated in Fig. 4, will initialize or initiate the execution of the algorithm, provided no other requirements or criteria, such as those described in greater detail below, must be met prior to initialization/initiation.

While the description above has thus far been primarily with respect to determining whether the catheter 16 is stationary based on data acquired by the ECU 22 from one or more sensors 54 mounted in or on the catheter 16 or located in close proximity thereto, the present disclosure is not meant to be so limited. Rather, in other exemplary embodiments, such data may be acquired from sources other than sensors 54.

For example, in one embodiment, the data may be acquired from the visualization, navigation, and/or mapping system 20. In an exemplary embodiment wherein the ECU 22 is part of the system 20, the ECU 22 is configured to monitor or track the movement of the catheter 16 and to store data relating to the movement or motion of the catheter in a storage medium associated with or accessible by the ECU 22. This data can be time averaged and analyzed by the ECU 22 to see if there is a trend in the data, and the information represented thereby, in particular. If there is little to no trend over the given window of time being analyzed, the ECU 22 can determine that the catheter is stationary. Accordingly, in an embodiment wherein the ECU 22 is configured to both determine whether to initialize or initiate the algorithm and to monitor and track the movement of the catheter, the ECU 22 is configured to acquire the data necessary to make a determination as to whether the catheter 16 is stationary from itself. Alternatively, whether or not the ECU 22 is part of the system 20, the data may be acquired from another component of the system 20, such as, for example, another processor in the system 20, and/or the sensors 18 disposed in or on the shaft 30 of the catheter 16.

In another exemplary embodiment, data representing motion or movement of the catheter 16 generated by the visualization, navigation, and/or mapping system 20 can be evaluated and the frequency component of motion of that data can be used. More particularly, correlation between a signal representative of catheter movement/motion and a respiration signal (which may comprise a thoracic impedance signal that changes during the course of respiratory activity) generated by, for example, the system 20, may also be indicative of motion resulting from commanded movement or movement resulting from respiratory activity. More specifically, if the catheter movement is highly correlated to the respiration signal, a determination can be made that the catheter movement results from respiratory activity and not commanded movement, and therefore, the catheter 16 can be deemed to be stationary. Conversely, if the catheter movement is not highly correlated to the respiration signal, a determination can be made that the catheter movement results from commanded movement, and therefore, the catheter 16 can be deemed to be not stationary. Whether the correlation is high or low can be evaluated using a predetermined correlation coefficient. Thus, depending on whether the computed correlation is above, meets, or falls below the predetermined threshold correlation coefficient, a determination can be made as to whether or not the catheter 16 is stationary. Accordingly, in an exemplary embodiment, the ECU 22 is configured to acquire these respective signals, to determine the correlation therebetween using techniques well known in the art, and to then make a determination with respect to whether the catheter 16 is stationary based on the correlation. Alternatively, another component of the visualization, navigation, and/or mapping system 20, such as, for example, a processor other than the ECU 22, may be configured to make the aforedescribed determination and to report the determination to the ECU 22.

In another exemplary embodiment, the coherence between typical commanded movements and the observed motion/movement of the catheter 16 can be determined and used in the determination of whether the catheter 16 is stationary. For example, if there is little coherence, a determination can be made that the movement of the catheter 16 is not the result of commanded movement, but rather is the result of respiratory activity, and therefore, the catheter 16 can be deemed to be stationary. Conversely, if there is a high degree of coherence, a determination can be made that the movement of the catheter 16 is the result of commanded movement, and therefore, the catheter 16 can be deemed to be not stationary. The coherence between the catheter 16 and other devices (e.g., catheters) known to be stationary could also be used such that if the movement of the catheter 16 is similar to that of a known-to-be-stationary devices and differs only by relative magnitude, a determination can be made that the catheter 16 is stationary. Accordingly, in an exemplary embodiment, the ECU 22 is configured to determine the coherence between the catheter 16 and either typical commanded movements or other known-to-be-stationary devices using techniques well known in the art, and to then make a determination with respect to whether the catheter 16 is stationary based on the coherence. Alternatively, another component of the visualization, navigation, and/or mapping system 20, such as, for example, a processor other than the ECU 22, may be configured to make the aforedescribed determination and to report the determination to the ECU 22.

Accordingly, those of ordinary skill in the art will appreciate that any number of techniques, including, without limitation, those described above (e.g., using information/data generated by sensors, a visualization, navigation, and/or mapping system, etc.), may be used to determine whether the catheter 16 is stationary.

As briefly described above, if the ECU 22 determines that the catheter 16 is stationary, it is configured to automatically initialize or initiate the execution of the motion compensation algorithm. Therefore, the ECU 22 is configured, as illustrated in Fig. 4, to set or update the parameters of the algorithm (step 108). Once the parameters of the algorithm are set or updated, the ECU 22 is configured to complete the execution of the algorithm in the usual manner described in greater detail above by computing a compensation signal (step 110), and then applying the compensation signal to the corresponding raw electrode location data (step 112) to generate compensated electrode location data. Conversely, if the ECU 22 determines that the catheter 16 is not stationary, the ECU 22 does not initialize or initiate the execution of the algorithm, and therefore, the algorithm is not executed and the raw location data is either not compensated for respiratory artifacts, or is compensated using a previously computed compensation signal.

The automatic initialization or initiation of the execution of the algorithm may be conducted any time that the catheter 16 is determined to be stationary. Accordingly, in such an embodiment, each time the ECU 22 determines that the catheter 16 is stationary, the algorithm is initialized/initiated and executed. The frequency at which the ECU 22 assesses whether the catheter 16 is stationary may vary.

For example, the ECU 22 may be configured to make the assessment in accordance with a predetermined sampling rate. In such an embodiment, the sampling rate may be set in a number of ways. For example, the sampling rate may be set prior to the system 10 or visualization, navigation, and/or mapping system 20 being used and is not adjustable. Alternatively, in another exemplary embodiment the sampling rate may be adjustable by the user. In the latter embodiment, the user may be able to set or adjust the sampling rate using, for example, the user interface 52 (best shown in Fig. 1). Alternatively, the ECU 22, or another component of the system 10 or visualization, navigation, and/or mapping system 20 that is electrically connected to and configured for communication with the ECU 22, may have a memory or other storage device, such as, for example, the memory 58 illustrated in Fig. 1, that contains a plurality of sampling rates. In such an embodiment, the ECU 22 may be configured to present the user with different sampling rate options on the display device 24, for example, and the user may then select the desired sampling rate using the user interface 52. Accordingly, the ECU 22 may acquire the sampling rate in a number of ways and/or from a number of sources, all of which are within the scope of the present disclosure.

Alternatively, the assessment of whether the catheter 16 is stationary may be made in accordance with the I/O cycle of the visualization, navigation, and/or mapping system 20, for example. More particularly, movement of the catheter 16 may be evaluated by the ECU 22 in accordance with a predetermined I/O cycle of the visualization, navigation, and/or mapping system 20, and then a determination as to whether the catheter 16 is stationary can be made based on that evaluation.

In yet another exemplary embodiment, rather than assessing whether the catheter 16 is stationary in accordance with a sampling rate or an I/O cycle, an assessment as to whether the catheter 16 is stationary is made only when a change in the position of the catheter 16 is sensed. Accordingly, the frequency and timing at which an assessment is made as to whether the catheter 16 is stationary may vary, and the present disclosure is not meant to be limited to any one particular technique.

While the description above has been with respect to an embodiment wherein the execution of a motion compensation algorithm is initialized or initiated whenever the catheter 16 is determined to be stationary, in another exemplary embodiment additional predetermined criteria must be met before the execution of the algorithm is initialized or initiated. For example, in addition to determining that the catheter 16 is stationary, in an exemplary embodiment, the ECU 22 is further configured to assess other predetermined criteria to determine when the execution of the algorithm is initialized/initiated (step 106). In other words, even though the ECU 22 may determine that the catheter 16 is stationary, it must also assess one or more criteria to determine whether or not to initiate the execution of the algorithm. Thus, in addition to the catheter 16 being stationary, one or more criteria must also be met in order to initialize/initiate the execution of the algorithm. Accordingly, in such an embodiment, whether the catheter 16 is stationary or not is but one factor to be taken into consideration in determining whether to initialize/initiate the execution of the algorithm.

In such an embodiment, the ECU 22 may take into consideration a number of different criteria, and therefore, the predetermined criteria that are assessed by the ECU 22 may comprise any number of criteria or combinations thereof. The criteria the ECU 22 may take into consideration may include, for example and without limitation, those described in greater detail below. It will be appreciated, however, that criteria other than those described with particularity herein may be additionally or alternatively taken into account in determining when or whether to initialize or initiate the execution of the algorithm and such criteria remain within the scope of the present disclosure.

One criterion the ECU 22 may take into consideration relates to the distance the catheter 16 has moved between the time the catheter 16 was most recently determined to be stationary and the time the algorithm was last executed. More particularly, the ECU 22 may be configured to only initialize or initiate the execution of the algorithm if the catheter 16 has moved at least a predetermined threshold distance since the algorithm was last executed. For example, in one embodiment, the ECU 22 is configured to initialize/initiate the execution of the algorithm only if the catheter 16 has moved at least five millimeters (5 mm) since the algorithm was last executed. It will be appreciated that this threshold distance may be less than or greater than 5 mm and so this particular value is provided for exemplary purposes only.

The threshold value may be set in a number of ways. In one embodiment, the threshold value is set prior to the system 10 or the visualization, navigation, and/or mapping system 20 being used and is not adjustable. Alternatively, in another exemplary embodiment the threshold value may be adjustable by the user. In the latter embodiment, the user may be able to set or adjust the threshold value using, for example, the user interface 52 (best shown in Fig. 1). Alternatively, the ECU 22, or another component of the system 10 or visualization, navigation, and/or mapping system 20 electrically connected to and configured for communication with the ECU 22, may have a memory or other storage device, such as, for example, the memory 58 illustrated in Fig. 1, that contains a plurality of threshold values. In such an embodiment, the ECU 22 may be configured to present the user with the different threshold value options on the display device 24, for example, and the user may then select the desired threshold value using the user interface 52. Accordingly, the ECU 22 may acquire the threshold value in a number of ways and/or from a number of sources, all of which are within the spirit and scope of the present disclosure.

The ECU 22 may acquire the distance the catheter 16 has moved since the algorithm was last executed in a number of ways. In an exemplary embodiment, the ECU 22 itself is configured to determine the distance moved based on positioning information it receives relating to the location of the catheter 16. This information may be received from the electrode 18 mounted on the catheter 16, for example, or may be received from another component of the system 10 or visualization, navigation, and/or mapping system 20 that is electrically connected to and configured for communication with the ECU 22 (*e.g.*, a visualization, navigation, and/or mapping system, such as, for example, the NavX system, in an embodiment wherein the ECU 22 is separate and distinct from the system 20). In either instance, the ECU 22 may be configured to maintain a log stored, for example, in a storage medium associated with or accessible by the ECU 22, that contains relative positions of the positioning electrode 18 (*e.g.*, x, y, z-coordinates) for each instance the catheter 16 is determined to be stationary and/or each instance algorithm is executed. The ECU 22 may then access the log and use the information stored therein to calculate the distance moved by the catheter 16. Accordingly, in such an embodiment, the ECU 22 is configured to calculate the distance moved by the catheter 16 and to use that calculated distance in evaluating whether or not to initialize or initiate the execution of the algorithm. In another exemplary embodiment, rather than the ECU 22 performing calculations to determine the magnitude of the distance moved by the catheter 16, the ECU 22 is configured to receive the magnitude of the distance moved from another component of the system 10 or the visualization, navigation, and/or mapping system 20.

In yet another exemplary embodiment, the moved distance may be acquired using a sensor, such as, for example, the sensor 54 described above (*e.g.*, a linear encoder). In such an embodiment, the sensor 54 is electrically connected to and configured for communication with the ECU 22. The sensor 54 is configured to generate electrical signals relating to the movement of the catheter 16, and the distance moved, in particular, which are communicated to the ECU 22 and used by the ECU 22 to determine whether or not the threshold distance has been met.

Another criterion the ECU 22 may take into consideration relates to the amount of time that has elapsed since the algorithm was last executed. More particularly, the ECU 22 may be configured to initialize or initiate the execution of the algorithm only if a predetermined amount of time has elapsed since the algorithm was last executed. For example, in one embodiment, the ECU 22 is configured to initialize/initiate the algorithm only if the algorithm has not been executed within the past five (5) minutes. It will be appreciated that this time value may be less than or greater than five minutes and so this particular value is provided for exemplary purposes only.

In an exemplary embodiment, the ECU 22 may be configured to maintain a log stored, for example, in a storage medium associated with or accessible by the ECU 22, that contains the relative time for each instance the catheter 16 is determined to be stationary and/or the algorithm was executed. Accordingly, each time the catheter 16 is deemed to be stationary and, in an exemplary embodiment, the algorithm executed, the relative time is recorded in the log. The ECU 22 may then access the log and use the information stored therein to calculate the elapsed time between the time at which the catheter 16 was most recently determined to be stationary and the time at which the algorithm was last executed. The ECU 22 can then determine whether or not to initialize/initiate the execution of the algorithm for the time at which the catheter 16 was most recently determined to be stationary. In an exemplary embodiment, the ECU 22 makes this determination by comparing the calculated elapsed time to a predetermined change-in-time value or threshold. If the change in time exceeds the threshold (or, in an exemplary embodiment, meets or exceeds the threshold), the execution of the algorithm is initialized or initiated, unless, of course, there are other criteria, such as those described above and below, that also must be met. If, however, the change in time falls below the threshold (or, in an exemplary embodiment, meets or falls below the threshold), the execution of the algorithm is not initialized or initiated.

As with the distance threshold described above, the time value may be set in a number of ways. In one embodiment, the value is set prior to the system 10 and/or the visualization, navigation, and/or mapping system 20 being used and is not adjustable. Alternatively, in another exemplary embodiment, the time value may be adjustable by the user. In the latter embodiment, the user may be able to set or adjust the value using, for example, the user interface 52 (best shown in Fig. 1). Alternatively, the ECU 22, or another component of the system 10 and/or the visualization, navigation, and/or mapping system 20 electrically connected to and configured for communication with the ECU 22, may have a memory or other storage device, such as, for example, the memory 58 illustrated in Fig. 1, that contains a plurality of time values.

In such an embodiment, the ECU 22 may be configured to present the user with the different time value options on the display device 24, for example, and the user may then select the desired time value using the user interface 52. Accordingly, the ECU 22 may acquire the time value in a number of ways and/or from a number of sources, all of which are within the spirit and scope of the present disclosure.

Yet another criterion the ECU 22 may take into consideration relates to whether the algorithm has been previously executed for the particular volumetric region of the anatomic structure within which the catheter 16 is disposed when the catheter 16 is most recently determined to be stationary. More particularly, a region-of-interest defined by the field generated by the visualization, navigation, and/or mapping system 20 (and, in an exemplary embodiment, the ECU 22 when the ECU 22 is part of the visualization, navigation, and mapping system 20) may be divided into distinct volumetric spaces (*e.g.*, the region-of-interest may be divided into a plurality of equal-sized cubes, for example) by the visualization, navigation, and mapping system 20. Each time the catheter 16 is determined to be stationary, the ECU 22 either determines in which volumetric space the catheter 16 is disposed, or receives such information from another component of the system 10, such as, for example, the visualization, navigation, and mapping system 20 when the ECU 22 is not part the system 20. In an exemplary embodiment, the ECU 22 may be configured to maintain a log stored, for example, in a storage medium associated with or accessible by the ECU 22, that contains the particular volumetric space corresponding to each instance the catheter 16 is determined to be stationary and/or each instance the algorithm is executed. The ECU 22 may then access the log and use the information stored therein to determine whether the algorithm has been previously executed for the volumetric space within which the catheter 16 is disposed when it is most recently determined to be stationary, and to then determine whether or not to initialize or initiate the execution of the algorithm.

Yet still another criterion the ECU 22 may take into consideration relates to whether or not there even is a respiration artifact, or at least one having a magnitude that would require compensation. A determination of whether or not there is a meaningful respiration artifact can be determined by evaluating data representing motion or movement of the catheter 16, and the frequency of motion thereof, in particular, that is generated by the visualization, navigation, and/or mapping system 20, for example. More particularly, correlation between a signal representative of catheter movement/motion and a respiration signal (which may comprise a thoracic impedance signal that changes during the course of respiratory activity) generated by, for example, the system 20, may be used to evaluate whether the respiration artifact is of such a magnitude that it would require compensation.

More specifically, if the catheter movement is highly correlated to the respiration signal, a determination can be made that the respiration artifact is of such a magnitude that it should be compensated for, and therefore, the execution of the algorithm should be initialized or initiated. Conversely, if the catheter movement is not highly correlated to the respiration signal, a determination can be made that the respiration artifact is of such a magnitude that it need not be compensated for, and therefore, the execution of the algorithm should not be initialized or initiated. Whether the correlation is high or low can be evaluated using a predetermined correlation coefficient. Thus, depending on whether the computed correlation is above, meets, or falls below the predetermined threshold correlation coefficient, a determination can be made as to whether or not the respiration artifact should be compensated for, and therefore, whether or not execution of the algorithm should be initialized or initiated. In an exemplary embodiment, the ECU 22 is configured to acquire these respective signals and to determine the correlation between the two using techniques that are well known in the art, and to then make the necessary determinations. Alternatively, another component of the visualization, navigation, and/or mapping system 20, such as, for example, a processor other than the ECU 22, may be configured to make the aforedescribed determination and to report the determination to the ECU 22.

In another exemplary embodiment, the coherence between typical commanded movements and the observed motion/movement of the catheter 16 can be determined and used in the determination of whether there is a meaningful respiration artifact, and therefore, whether or not the execution of the algorithm should be to initialized or initiated. For example, if there is little coherence, a determination can be made that the respiration artifact is of such a magnitude that it should be compensated for, and therefore, the execution of the algorithm should be initialized or initiated. Conversely, if there is a high degree of coherence, a determination can be made that the respiration artifact is of such a magnitude that it need not be compensated for, and therefore, the execution of the algorithm should not be initialized or initiated. The coherence between the catheter 16 and other devices (*e.g.*, catheters) known to be stationary could also be used such that if the movement of the catheter 16 is similar to that of a known-to-be-stationary devices and differs only by relative magnitude, a determination can be made that the respiration artifact is of such a magnitude that it should be compensated for, and therefore, the execution of the algorithm should be initialized or initiated, and vice versa. Accordingly, in an exemplary embodiment, the ECU 22 is configured to determine the coherence between the catheter 16 and either typical commanded movements or other known-to-be-stationary devices using techniques well known in the art, and to then make the necessary determinations. Alternatively, another component of the visualization, navigation, and/or mapping system 20, such as, for example, a processor other than the ECU 22, may be configured to make the aforedescribed determination and to report the determination to the ECU 22.

In an exemplary embodiment, the ECU 22 is configured to take only one of the above criteria into consideration. However, in other exemplary embodiments, any combination of more than one or all of the criteria may be taken into consideration by the ECU 22 in determining whether or not to initialize/initiate the algorithm. Likewise, the criteria may be considered in a linear fashion where a threshold must be exceeded, or may be considered in a sliding scale. For example, if a long time has passed since the motion compensation algorithm was initiated, it may not be necessary for the catheter 16 to have moved. On the other hand, if only a short time has passed, a larger movement by catheter 16 may be required. If an intermediate length of time has passed, an intermediate movement may be required. Two or more criteria may be considered in this fashion. For example, if a short time has passed, but the catheter 16 has been moved to a new location and the respiration artifact is large, this may trigger the algorithm.

With reference to Figs. 4 and 6-9, the automatic initialization or initiating of the execution of a motion compensation algorithm for an automated medical device guidance system will be described. Before proceeding to a detailed description of how the execution of the algorithm is automatically initialized or initiated in such systems, a brief overview (for context) of two exemplary automated medical device guidance systems - one robotic-based system and one magnetic-based system - will first be provided. Automated medical device guidance systems may take a number of forms, such as, for example, robotic-based systems and magnetic-based systems. Each of these exemplary types of automated systems will be briefly described in turn below.

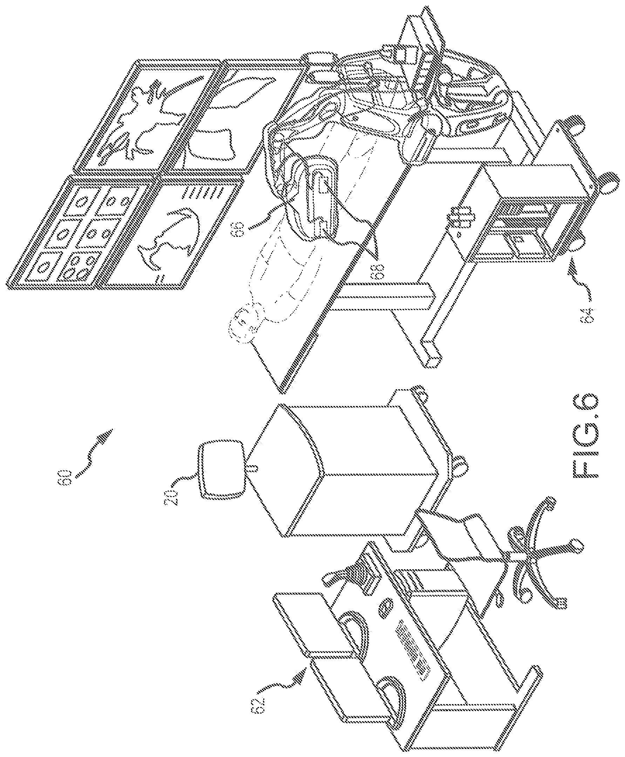

Figure 6 illustrates an exemplary robotic-based system 60 for manipulating a medical device, such as, for example, the catheter 16. The robotic-based system 60 permits control of translation, distal bending, and virtual rotation of the catheter 16, and therefore, provides the user with a type of control similar to that provided by manually-operated systems, but also allows for repeatable, precise, and dynamic movements. A clinician may identify target locations (potentially forming a path) on an image of an anatomic structure. The robotic-based system 60 relates these digitally selected points to positions within the patient's actual/physical anatomy, and may thereafter command and control the movement of the catheter 16 to the defined positions wherein the clinician or the robotic-based system 60 can perform a desired diagnostic and/or therapeutic function. Referring to Fig. 6, the robotic-based system 60 may include an input control system 62, an electronic control system 64, and a manipulator assembly 66 for operating a device cartridge 68.

The input control system 62 is provided to allow the clinician to interact with the robotic-based system 60 to control movement of the catheter 16. The input control system 62 may include, for example and without limitation, instrumented traditional catheter handle controls, oversized catheter models, instrumented user-wearable gloves, touch screen display monitors, 2-D input devices, 3-D input devices, spatially detected styluses, and traditional joysticks. These input devices may be configured to directly control the movement of the catheter 16, or may be configured, for example, to manipulate a target or cursor on an associated display.

The electronic control system 64 is configured to translate (*i.e.*, interpret) inputs (*e.g.*, motions) of the user at an input device (*e.g.*, the user input system 62) or from another source into a resulting movement of the catheter 16. The electronic control system 64 issues commands to the manipulator assembly 66 (*i.e.*, to the actuation units - electric motors) to move or bend the catheter 16 to prescribed positions and/or in prescribed ways, all in accordance with the received user input and a predetermined programmed operating strategy. The electronic control system 64 may include one or more stand-alone microprocessors or application specific integrated circuits (ASICs). Alternatively, the electronic control system 64 may form a part of the visualization, navigation, and/or mapping system 20 and/or the ECU 22 described above.

The manipulator assembly 66 is configured to maneuver the catheter 16 in response to commands from the electronic control system 64. The assembly 66 may cause translational movement such as advancement or withdrawal of the catheter 16 and effect deflection of the distal end 34 of the catheter 16 and/or rotation or virtual motion. The assembly 66 may include conventional actuation mechanisms (*e.g.*, a plurality of electric motor and lead screw combinations) for linearly actuating one or more control members (*e.g.*, steering wires) associated with the medical device for achieving the above-described translation, deflection, and/or rotation (or virtual rotation).

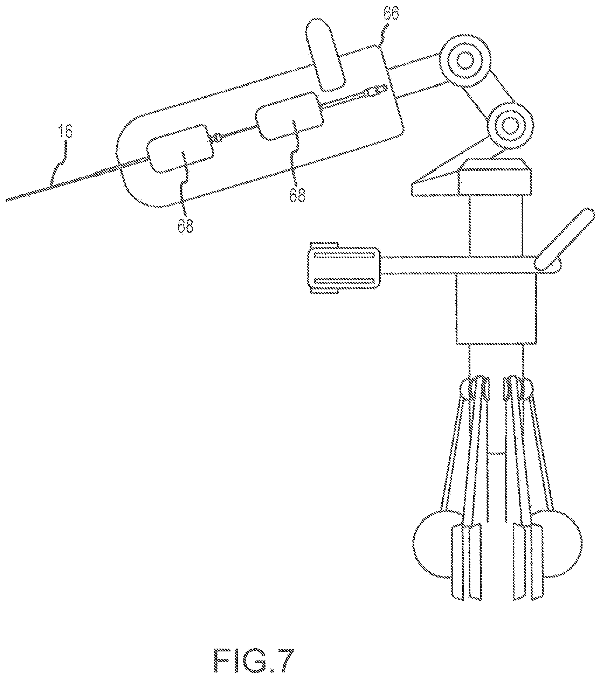

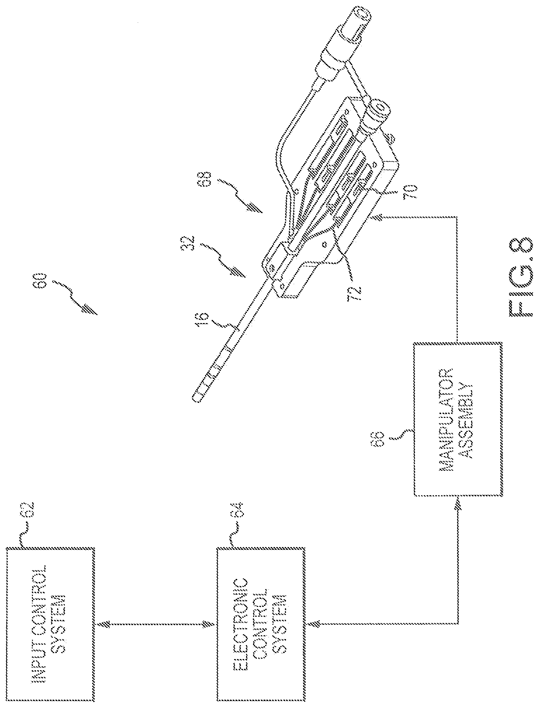

With reference to Figs. 7 and 8, the device cartridge 68 is provided to translate movement of elements in the manipulator assembly 66 to the catheter 16. The cartridge 68 receives and retains the proximal end 32 of the catheter 16. The cartridge 68 may include sliding blocks 70 each coupled to a corresponding steering wire 72 so as to permit independent tensioning of each wire 72. Movement of the blocks 70 is controlled by the manipulator assembly 66 to cause tensioning of the wires 72 and thereby affect translation, deflection, and rotation of the catheter 16.

A more complete description of various elements of a robotic-based system, such as the robotic-based system 60, may be found in the following patent applications that are incorporated herein by reference: U.S. Patent Application No. 12/933,063 filed September 16, 2010 and titled "Robotic Catheter System Input Device"; U.S. Patent Application No. 12/751,843 filed March 31, 2010 and titled "Robotic Catheter System"; U.S. Patent Application No. 12/347,842 filed December 13, 2008 and titled "Robotic Catheter Rotatable Device Cartridge"; U.S. Patent Application No. 12/347,826 filed December 31, 2008 and titled "Robotic Catheter Manipulator Assembly"; U.S. Patent Application No. 12/347,811 filed December 31, 2008 and titled "Robotic Catheter System"; U.S. Patent Application No. 12/347,442 filed December 31, 2008 and titled "Model Catheter Input Device" and International Patent Application No. PCT/US2009/038597 filed March 29, 2009 and titled "Robotic Catheter System With Dynamic Response" (published as WO 2009/120982).

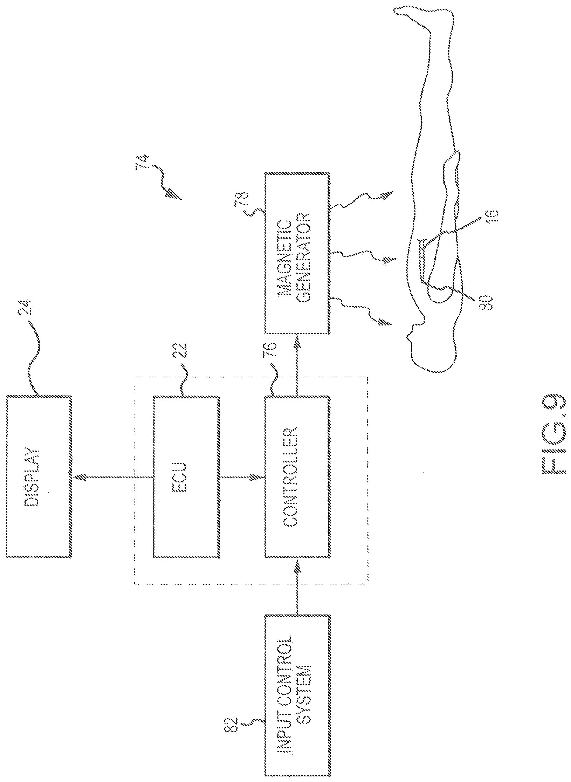

With reference to Fig. 9, a magnetic-based automated guidance system 74 will be briefly described. In one exemplary embodiment, one or more externally generated magnetic fields produced by one or more electromagnets are used to move, guide, and/or steer a magnetically-tipped medical device, such as the catheter 16, through a patient's body. The externally generated magnetic fields exert a desired torque on the catheter 16 to cause the position of the catheter 16 to be manipulated in a desired way (e.g., advance, retract, bend, rotate, speed up, slow down, etc.). Accordingly, as with the robotic-based system 60 described above, the magnetic fields may be used to control the movement of the catheter 16 and/or to allow the system 10 to carry out therapeutic, diagnostic, or other activities at given locations within the patient's body 14. A full description of a magnetic-based catheter system, such as the magnetic-based system 74, is set forth in U.S. Patent No. 6,507,751 entitled "Method and Apparatus Using Shaped Field of Repositionable Magnet to Guide Implant," and U.S. Published Patent Application No. 2007/0016006 A1 entitled "Apparatus and Method for Shaped Magnetic Field Control for Catheter, Guidance, Control, and Imaging,".

To summarize, however, in an exemplary embodiment, the magnetic-base automated guidance system 74 includes a controller or control system 76 that may be part of the ECU 22 or may be a separate and distinct component that is electrically connected to and configured for communication with the ECU 22. In either instance, the controller 76 is configured to control the movement of the catheter 16 by adjusting the strength of the magnetic fields generated by one or more electromagnets. As with the robotic-based system 60 described above, the magnetic-based system 74 provides the ability for precise and dynamic automated control in, for example, diagnostic, therapeutic, mapping, and ablative procedures. In an exemplary embodiment, the magnetic-based system 74 includes somewhat similar structure to that of the robotic-based system 60 to effect the movement of the catheter 16. For example, the magnetic-based system 74 may comprise a medical device manipulator assembly that includes, in part, one or more external magnetic field generators 78 configured to create the magnetic field(s) required to induce the movement of the catheter 16, and a magnetic element 80 mounted thereon or therein. The magnetic-based system 74 may further comprise support structures and the like to support the catheter 16.

As with the robotic-based system 60, the magnetic-based system 74 may further include a human input device and control system ("input control system") 82, which may include a joystick and related controls with which a physician/clinician may interact to control the manipulation the catheter 16. In one exemplary embodiment, the magnetic-based system 74 is configured such that the physician or clinician may input a command for the catheter 16 to move in a particular way. The magnetic-based system 74 processes that input and adjusts the strength and/or orientation of the external magnetic fields to cause the catheter 16 to move as commanded. The magnetic-based system 74 may also still further include an electronic control system, which, as with the electronic control system 64 of the robotic-based system 60 described above, may consist of or include a controller, such as, for example, the controller 76, that translates motions of the physician/clinician at the input device 82 into a resulting movement of the catheter 16. Finally, in an exemplary embodiment, the magnetic-based system 74 may further include or be a constituent part of the visualization, navigation, and/or mapping system 20, to provide the clinician/physician with real-time or near-real-time positioning information concerning the catheter 16 and various types of anatomical maps, models, and/or geometries of the anatomic structure of interest, for example.

With reference now to Fig. 4, the automatic initialization or initiating of the execution of the motion compensation algorithm for an automated medical device system, such as, for example, the robotic and magnetic-based systems 60, 74 described above, will be described. As with the automatic initialization/initiating of the algorithm for manually-operated or user-controlled systems, in an exemplary embodiment, the ECU 22 is configured to acquire data representing information relating to movement of the catheter 16.

The ECU 22 may receive the electrical signal(s) from one or more of a number of sources, including, as was described in great detail above, sensors mounted in/on the catheter 16 or located in close proximity thereto, and other components of the system 10, such as, for example, the visualization, navigation, and/or mapping system 20. The descriptions set forth above with respect to the acquiring of data representing information relating to movement of the catheter 16 apply here with equal weight, and therefore, will not be repeated but rather are incorporated here by reference.

Another source from which the ECU 22 may acquire data is the controller of the automated medical guidance system. More particularly, in an exemplary embodiment wherein the controller of the automated medical device guidance system (e.g., the electronic control system 64 of the robotic-based system 60 and/or the controller 76 of the magnetic-based system 74) is separate and distinct from the ECU 22, the ECU 22 is configured to receive electrical signal(s) containing data representing information relating to commanded movement of the catheter 16 from the controller of the automated medical device guidance system. More particularly, because the controller controls the commanded movement of the catheter 16, it necessarily knows when the catheter 16 is experiencing commanded movement. As such, the electrical signal(s) received by the ECU 22 may be an electrical signal(s) provided by the controller indicating that the controller is or is not causing the catheter 16 to move.

In an exemplary embodiment, the electrical signal(s) is provided by the controller only when the catheter 16 is being commanded to move such that no signal is provided when the catheter 16 is not experiencing commanded movement. For example, a digital "high" signal (*i.e.*, a binary "1" signal) may represent that the controller is causing the catheter 16 to move, and therefore, the catheter 16 is not stationary, and a digital "low" signal (*i.e.*, a binary "0" signal) may represent that the controller is not commanding movement of the catheter 16, and therefore, the catheter 16 is stationary. It will be appreciated that the opposite scheme is also applicable whereby a digital "low" may represent that the controller is commanding movement of the catheter, and therefore, the catheter 16 is not stationary, and a digital "high" represents that the controller is not commanding movement of the catheter, and therefore, the catheter 16 is stationary. Accordingly, in such an embodiment, the presence or absence of the electrical signal(s) is indicative of whether the catheter 16 is stationary.

In another exemplary embodiment, the electrical signal(s) may not simply be "high" or "low" signals, but rather may represent information that can be interpreted by the ECU 22 to determine whether or the controller is commanding movement of the catheter 16. These signals may comprise, for example, the actual commands sent to the manipulator assembly of the automated medical device guidance system (*e.g.*, the manipulator assembly 66 of the robotic-based system 60 and/or the magnetic generator 78 of the magnetic-based system 74), or other electrical signals generated by the controller representing information relating to the commanded movement of the catheter 16.

In yet another exemplary embodiment wherein the controller of the automated medical device guidance system comprises the ECU 22, the ECU 22 itself controls the movement of the catheter 16, and therefore, effectively acquires data representing information relating to commanded movement of the catheter 16 from itself. Accordingly, for purposes of this disclosure and in this context, the term "receives" is meant to encompass the instance wherein the ECU 22 itself controls the movement of the catheter 16, and therefore, inherently receives information relating to the commanded movement of the catheter 16, in addition to the instance where the ECU 22 receives signals in the traditional sense.

In yet still another exemplary embodiment, the automated medical device guidance system may further include sensors configured to detect or sense movement of the manipulator assembly (*e.g.*, the manipulator assembly 66 of the robotic-based system 60 and/or the magnetic generator 78 of the magnetic-based system 74), or the constituent components thereof, such as, for example, actuation units or mechanisms (*e.g.*, motors, motor/lead screw combinations, and the like). Accordingly, in an exemplary embodiment, one or more sensors, such as, for example, conventional rotary or linear encoders, are disposed proximate the manipulator assembly, and the actuation units or mechanisms thereof, in particular. These sensors are electrically connected to, and configured for communication with, the ECU 22, and therefore, the electrical signals generated by the sensors containing data representing information relating to the commanded movement of the catheter 16 are received by the ECU 22.

Thus, as with user-controlled systems, in automated medical device guidance systems the ECU 22 is programmed or configured to process data representing information relating to movement of the catheter 16 and to determine whether the catheter 16 is stationary based on that data and the information it represents, in particular. Accordingly, the ECU 22 is configured to extract the information represented by the acquired data and to determine, based on the information, whether the catheter 16 is stationary.

In an exemplary embodiment, the ECU 22 is configured to make such a determination based solely on the information represented by the acquired data. Accordingly, in such an embodiment, if the information indicates that there is currently commanded movement of the catheter 16, the ECU 22 will determine that the catheter 16 is not stationary, and therefore, as illustrated in Fig. 4, will not initiate the execution of the motion compensation algorithm. On the other hand, if the information indicates that the catheter 16 is not currently experiencing commanded movement, the ECU 22 will determine that the catheter 16 is stationary, and therefore, as illustrated in Fig. 4, will initiate the execution of the motion compensation algorithm, provided no other requirements or criteria, such as those described in greater detail above and below, must be met prior to execution of the algorithm.

In another exemplary embodiment, the ECU 22 is configured to make the determination based on the information represented by the acquired data, as well as one or more predetermined conditions. These predetermined conditions may include, for example, that there has been no commanded movement within a predetermined period of elapsed time. For instance, the ECU 22 may be programmed such that it will only determine that the catheter 16 is stationary if there has not been commanded movement of the catheter 16 over the past ten (10) seconds. Accordingly, in such an embodiment, if the information indicates that there has been commanded movement of the catheter within a predetermined period of elapsed time, the ECU 22 will determine that the catheter is not stationary, and therefore, as illustrated in Fig. 4, will not initialize or initiate the execution of the algorithm. On the other hand, if the information indicates that there has not been commanded movement of the catheter 16 within the predetermined period of elapsed time, the ECU 22 will determine that the catheter 16 is stationary, and therefore, as illustrated in Fig. 4, will initialize or initiate the execution of the algorithm, provided no other requirements or criteria, such as those described in greater detail below, must be met prior to execution.

Accordingly, as described above, if the ECU 22 determines that the catheter 16 is stationary, it is configured to automatically initialize or initiate the execution of the motion compensation algorithm. Therefore, the ECU 22 is configured, as illustrated in Fig. 4, to set or update the parameters of the algorithm (step 108). Once the parameters of the algorithm are set or updated, the ECU 22 is configured to complete the execution of the algorithm in the usual manner described in greater detail above by computing a compensation signal (step 110), and then applying the compensation signal to the corresponding raw electrode location data (step 112) to generate compensated electrode location data. Conversely, if the ECU 22 determines that the catheter 16 is not stationary, the ECU 22 does not initialize or initiate the execution of the algorithm, and therefore, the algorithm is not executed and the raw location data is not compensated for respiratory artifacts.

As with the manually-operated systems described above, the automatic initialization or initiation of the execution of the algorithm may be conducted any time that the catheter 16 is determined to be stationary. Accordingly, in such an embodiment, each time the ECU 22 determines that the catheter 16 is stationary; the algorithm is initialized/initiated and executed. The description set forth above with respect to the frequency at which the ECU 22 assesses whether the catheter 16 is stationary may applies here with equal weight, and therefore, will not be repeated but rather is incorporated here by reference.

While the description above has been with respect to an embodiment wherein the execution of a motion compensation algorithm is initialized or initiated whenever the catheter 16 is determined to be stationary, in another exemplary embodiment additional predetermined criteria must be met before the execution of the algorithm is initialized or initiated. For example, in addition to determining that the catheter 16 is stationary, in an exemplary embodiment, the ECU 22 is further configured to assess other predetermined criteria to determine when the execution of the algorithm is initialized/initiated (step 106). In other words, even though the ECU 22 may determine that the catheter 16 is stationary, it must also assess one or more criteria to determine whether or not to initiate the execution of the algorithm. Thus, in addition to the catheter 16 being stationary, one or more criteria must also be met in order to initialize/initiate the execution of the algorithm. Accordingly, in such an embodiment, whether the catheter 16 is stationary or not is but one factor to be taken into consideration in determining whether to initialize/initiate the execution of the algorithm.

In such an embodiment, the ECU 22 may take into consideration a number of different criteria, and therefore, the predetermined criteria that are assessed by the ECU 22 may comprise any number of criteria or combinations thereof. The criteria may be the same as those described above with respect to manually-operated systems. As such, the description above relating to the use of predetermined criteria applies here with equal weight, and therefore, will not be repeated here but rather is incorporated by reference.

It will be appreciated that in addition to the structure of the system 10 described above, another aspect of the present disclosure is a method for automatically initializing or initiating the execution of a motion compensation algorithm. In an exemplary embodiment, and as described above, the ECU 22 is configured to perform the methodology. However, in other exemplary embodiments, the ECU 22 is configured to perform some, but not all, of the methodology. In such an embodiment, another component or components that is/are part of the system 10 or the visualization, navigation, and/or mapping system 20, or that is/are configured for communication with the system 10 or the visualization, navigation, and/or mapping system 20, and the ECU 22 thereof, in particular, is/are configured to perform some of the methodology.

With reference to Fig. 4, in an exemplary embodiment the method in its most general form includes a step 100 of acquiring data representing information relating to movement of a medical device, such as, for example, the catheter 16 described above. In an exemplary embodiment, the acquiring step 100 comprises receiving electrical signal(s) generated by a sensor associated with, or located in close proximity to, the medical device and electrically connected to the ECU 22, such as, for example, the sensors 54 described above. In such an embodiment, the sensor is configured to detect movement of the medical device and to generate one or more electrical signals representative of such movement, or, in certain embodiments, the lack thereof. In another exemplary embodiment, the acquiring step 100 comprises receiving electrical signals from a controller of an automated medical device guidance system, such as, for example, the automated medical device guidance systems described above. In still another exemplary embodiment, the acquiring step 100 comprises receiving electrical signals from one or more sensors associated with a manipulator assembly of the automated medical device guidance system, such as, for example, the manipulator assembly 66 (and the constituent components thereof, in particular) of the robotic-based system 60 described above. In still another exemplary embodiment, the acquiring step 100 comprises receiving data from a component of a visualization, navigation, and/or mapping system, such as, for example, the system 20 described above.

In an exemplary embodiment, the method further comprises a step 102 of determining whether the medical device is stationary based on the information represented by the acquired data.

As illustrated in Fig. 4, the method further comprises a step 104 of automatically initializing or initiating the execution of the motion compensation algorithm in response to a determination that the medical device is stationary. If the algorithm is initialized or the execution thereof is initiated, the method further comprises a step 108 of setting or updating the parameters of the algorithm, followed by the completion of the execution of the algorithm in the usual manner described in greater detail above by performing a step 110 of computing a compensation signal, and then a step 112 of applying the compensation signal to the corresponding raw electrode location data to generate compensated electrode location data. If, however, the medical device is not stationary, the method returns to the receiving step 100 and is repeated.

In an exemplary method not forming part of the claimed invention, the method further comprises a step 106 of assessing predetermined criteria to determine when to initialize or initiate the execution of the algorithm. In such an embodiment, the initiating step 104 comprises automatically initiating the algorithm when the medical device is determined to be stationary and certain of the predetermined criteria are met. As was described above, if the algorithm is initialized or the execution thereof is initiated, the method further comprises the step 108 of setting or updating the parameters of the algorithm, followed by the completion of the execution of the algorithm in the usual manner described in greater detail above by performing the step 110 of computing a compensation signal, and then the step 112 of applying the compensation signal to the corresponding raw electrode location data to generate compensated electrode location data. If, however, the medical device is not stationary, the method returns to the receiving step 100 and is repeated.

As described above, one exemplary criterion that may be taken into consideration relates to the distance the medical device has moved between the time at which the medical device was most recently determined to be stationary and the time at which the algorithm was last executed. More particularly, the algorithm may only be initialized or initiated if the medical device has moved at least a predetermined threshold distance since the algorithm was last executed. Accordingly, in an exemplary embodiment, the assessing step 106 comprises a substep of acquiring the distance the medical device has moved since the algorithm was last executed. In an exemplary embodiment, the substep comprises acquiring location or position coordinates of the medical device corresponding to when algorithm was last executed, acquiring current location or position coordinates of the medical device, and then calculating the distance moved based thereon. Alternatively, the substep may simply comprise receiving the value of the distanced moved as opposed to calculating the distance.

In an exemplary embodiment, the assessing step 106 comprises a second substep of comparing the distance moved to the predetermined threshold distance. If the distance moved exceeds the threshold (or, in an exemplary embodiment, meets or exceeds the threshold), the initiating step 104 is performed, unless, of course, there are other criteria, such as those described below, that also must be met. If, however, the distance moved falls below the threshold (or, in an exemplary embodiment, meets or falls below the threshold), the initiating step is not performed, and the method starts over again at receiving step 100.

Another exemplary criterion that may be taken into consideration relates to the amount of time that has elapsed since the algorithm was last executed. More particularly, the algorithm may only be initialized or initiated if a predetermined amount of time has elapsed since the algorithm was last executed. Accordingly, in an exemplary embodiment, the assessing step 106 comprises a substep of acquiring the relative time at which the algorithm was last executed. In an exemplary embodiment, the substep comprises calculating the change in time between the time at which the medical device was most recently determined to be stationary and the time at which the algorithm was last executed. Alternatively, the substep may simply comprise receiving the change in time as opposed to calculating the change in time.

In an exemplary embodiment, the assessing step 106 comprises a second substep of comparing the change in time with the predetermined change-in-time value or threshold. If the change in time exceeds the threshold (or, in an exemplary embodiment, meets or exceeds the threshold), the initiating step 104 is performed, unless, of course, there are other criteria, such as those described above and below, that also must be met. If, however, the change in time falls below the threshold (or, in an exemplary embodiment, meets or falls below the threshold), the initiating step 104 is not performed, and the method starts over again at receiving step 100.

Yet another exemplary criterion that may be taken into consideration relates to whether the algorithm has been previously executed for the particular volumetric region of the anatomic structure within which the medical device is disposed when the device is most recently determined to be stationary. More particularly, execution of the algorithm may only be initialized or initiated if the algorithm has not previously been executed for the particular volumetric space within which the medical device is disposed when it is most recently determined to be stationary. Accordingly, in an exemplary embodiment, the assessing step 106 comprises a substep of determining the volumetric space the medical device is disposed within when it is determined to be stationary. The assessing step further comprises a substep of comparing the volumetric space within which the medical device is disposed with those volumetric spaces for which the algorithm has been previously executed. In an exemplary embodiment, the substep comprises looking up the volumetric space within which the medical device is disposed in a log containing the volumetric spaces for which the algorithm has been previously executed.

If the algorithm has not been previously executed for the particular volumetric space within which the medical device is disposed, the initiating step 104 is performed, unless, of course, there are other criteria, such as those described above, that also must be met. If, however, the algorithm has been previously executed for the volumetric space within which the medical device is disposed, the initiating step 104 is not performed, and the method starts over again at receiving step 100.

Yet still another criterion that may be taken into consideration relates to whether or not there even is a respiration artifact, or at least one having a magnitude that would require compensation. A determination of whether or not there is a meaningful respiration artifact can be determined in the manner described above, the description of which will not be repeated but rather is incorporated here by reference. Accordingly, in an exemplary embodiment, the assessing step 106 comprises a substep of determining whether or not there is a respiration artifact and if so, whether or not it is of such a magnitude that it should be compensated for.

If it is determined that there is, in fact, a respiration artifact that should be compensated for, the initiating step 104 is performed, unless, of course, there are other criteria, such as those described above, that also must be met. If, however, it is determined that there is not a respiration artifact that should be compensated for, the initiating step 104 is not performed, and the method starts over again at receiving step 100.

It will be appreciated that additional functionality described in greater detail above with respect to the system 10 and the visualization, navigation, and/or mapping system 20, and the ECU 22 thereof, in particular, may also be part of the inventive methodology. Therefore, to the extent such functionality has not been expressly described with respect to the methodology; the description thereof above is incorporated herein by reference.

It should be understood that the system 10 and the visualization, navigation, and/or mapping system 20 as described above may include conventional ECU 22 known in the art, capable of executing pre-programmed instructions stored in an associated memory, all performing in accordance with the functionality described herein. It is contemplated that the methods described herein will be programmed in a preferred embodiment, with the resulting software being stored in an associated memory and where so described, may also constitute the means for performing such methods. Implementation of the invention, in software, in view of the foregoing enabling description, would require no more than routine application of programming skills by one of ordinary skill in the art. Such a system may further be of the type having both ROM, RAM, a combination of non-volatile and volatile (modifiable) memory so that the software can be stored and yet allow storage and processing of dynamically produced data and/or signals.

Although only certain embodiments have been described above with a certain degree of particularity, those skilled in the art could make numerous alterations to the disclosed embodiments without departing from the scope of this disclosure. Joinder references (e.g., attached, coupled, connected, and the like) are to be construed broadly and may include intermediate members between a connection of elements and relative movement between elements. As such, joinder references do not necessarily infer that two elements are directly connected/coupled and in fixed relation to each other. Additionally, the terms "electrically connected" and "in communication" are meant to be construed broadly to encompass both wired and wireless connections and communications. It is intended that all matter contained in the above description or shown in the accompanying drawings shall be interpreted as illustrative only and not limiting. Changes in detail or structure may be made without departing from the invention as defined in the appended claims.

**Claims**

1. A system for automatically initializing or initiating a motion compensation algorithm for compensating respiration, cardiac activity and/or other cardiac motion artifacts, comprising
   a medical device (16) adapted for a commanded movement including a catheter comprising one or more positioning sensors (18) for determining raw uncompensated location coordinates of the catheter;
   a processing apparatus (22) configured to:

   acquire data representing information relating to a commanded movement of the catheter disposed within an anatomic structure;
   determine whether said catheter is in an anatomically stable position based on said information represented by said acquired data; and
   automatically initiate the execution of the motion compensation algorithm in response to a determination that said catheter is in an anatomically stable position for correcting the raw uncompensated location coordinates.

2. The system of claim 1 wherein said processing apparatus (22) is configured to acquire said data from one of:

   an automated medical device guidance system (64, 76);
   a sensor (54) configured to detect movement of said medical device (16); and
   a visualization, navigation, and/or mapping system (20).

3. The system of claim 1 or 2 wherein said processing apparatus (22) is configured to determine if said medical device (16) is in an anatomically stable position by determining whether there has been commanded movement of said medical device (16) within a predetermined period of elapsed time.

4. The system of any one of claims 1 to 3 wherein said processing apparatus (22) is further configured to assess predetermined criteria to determine when to initiate the execution of the motion compensation algorithm, and to initiate the execution of the algorithm when said processing apparatus (22) determines that said medical device (16) is in an anatomically stable position and certain of said predetermined criteria are met.

5. The system of claim 4 wherein said processing apparatus is configured to initiate the execution of the motion compensation algorithm only when in addition at least one of the following of said predetermined criteria are met:

   when a distance moved by said medical device since a most recent execution of the algorithm exceeds a predetermined distance threshold;
   when a predetermined amount of time has elapsed since a most recent execution of the algorithm;
   when a commanded movement of said medical device (16) results in a distance moved by said medical device within a predetermined period of elapsed time that exceeds a predetermined distance threshold;
   when said medical device (16) is determined to be disposed within a volumetric region of an anatomic structure for which the algorithm has not been previously executed; and
   when said processing apparatus (22) determines that a respiration artifact exists in location data corresponding to a location of said medical device, and said respiration artifact has such a magnitude that compensation is required.

6. The system of any one of claims 2 to 5 wherein the sensor (54) is electrically connected to said processing apparatus (22) and configured to generate an electrical signal (50) containing said data representing information relating to commanded movement of said medical device (16), and said processing apparatus (22) is configured to receive said electrical signal (50) from said sensor.

7. The system of any one of claims 2 to 6 wherein said sensor (54) comprises one of an optical sensor and a magnetic sensor.

8. The system of any one of claims 2 to 7 wherein said medical device (16) comprises a shaft (30) having at least one marker (56) disposed thereon, said sensor (54) configured to detect movement of said at least one marker (56) and to generate said electrical signal (50) in response thereto.

9. The system of any one of claims 1 to 8 wherein said medical device (16) comprises a sheath.

**Patentansprüche**

1. System zum automatischen Initialisieren oder Initiieren eines Bewegungskompensationsalgorithmus zur Kompensation von Atmung, Herzaktivität und/oder anderen Herzbewegungsartefakten, mit einer medizinischen Vorrichtung (16), die angepaßt ist zu einer befohlenen Bewegung mit einem Katheter, der einen oder mehrere Positionssensoren (18) aufweist zum Bestimmen von unkompensierten Rohortskoordinaten des Katheters;
   einer Verarbeitungsvorrichtung (22), die konfiguriert ist zum:

   Erfassen von Daten, die Information darstellen, die eine befohlene Bewegung des Katheters betrifft, der sich innerhalb einer anatomischen Struktur befindet;
   Bestimmen, ob der Katheter in einer anatomisch stabilen Position ist, basierend auf der Information, die die erfassten Daten darstellt; und
   automatischen Initiieren der Ausführung des Bewegungskompensationsalgorithmus in Antwort auf eine Bestimmung, dass der Katheter in einer anatomisch stabilen Position ist zur Korrektur der unkompensierten Rohortskoordinaten.

2. System nach Anspruch 1, bei dem die Verarbeitungsvorrichtung (22) konfiguriert ist zum Erfassen der Daten von:

   einem automatisierten medizinischen Vorrichtungsführungssystem (64, 76); oder
   einem Sensor (54), der konfiguriert ist zum Detektieren der Bewegung der medizinischen Vorrichtung (16); oder
   einem Visualisierungs-, Navigations- und/oder Abbildungssystem (20).

3. System nach Anspruch 1 oder 2, bei dem die Verarbeitungsvorrichtung (22) konfiguriert ist zum Bestimmen, ob die medizinische Vorrichtung (16) in einer anatomisch stabilen Position ist, indem bestimmt wird, ob innerhalb einer vorbestimmten Periode einer verstrichenen Zeit eine Bewegung der medizinischen Vorrichtung (16) befohlen wurde.

**4.** System nach einem der Ansprüche 1 bis 3, bei dem die Verarbeitungsvorrichtung (22) ferner konfiguriert ist zur Beurteilung vorbestimmter Kriterien, um zu bestimmen, wann die Ausführung des Bewegungskompensationsalgorithmus zu initiieren ist, und die Ausführung des Algorithmus zu initiieren, wenn die Verarbeitungsvorrichtung (22) bestimmt, dass die medizinische Vorrichtung (16) in einer anatomisch stabilen Position ist und bestimmte der vorbestimmten Kriterien erfüllt sind.

**5.** System nach Anspruch 4, bei dem die Verarbeitungsvorrichtung konfiguriert ist zum Initiieren der Ausführung des Bewegungskompensationsalgorithmus, nur wenn zusätzlich mindestens eine der folgenden der vorbestimmten Kriterien erfüllt ist:

wenn ein Abstand, um den sich die medizinische Vorrichtung, seit der letzten Ausführung des Algorithmus bewegt hat, einen vorbestimmten Distanzschwellenwert überschreitet;
wenn eine vorbestimmte Zeit seit der letzten Ausführung des Algorithmus verstrichen ist;
wenn eine befohlene Bewegung der medizinischen Vorrichtung (16) einen Abstand zur Folge hat, um den sich die medizinische Vorrichtung innerhalb einer vorbestimmten Periode einer verstrichenen Zeit bewegt hat, der einen vorbestimmten Distanzschwellenwert überschreitet;
wenn die medizinische Vorrichtung (16) als innerhalb einer Volumenregion der anatomischen Struktur bestimmt wird, für die der Algorithmus vorher nicht ausgeführt worden ist; und
wenn die Verarbeitungsvorrichtung (22) bestimmt, dass ein Atmungsartefakt in den Ortsdaten existiert, die einem Ort der medizinischen Vorrichtung entsprechen, und das Atmungsartefakt eine derartige Größe hat, dass eine Kompensation erforderlich ist.

**6.** System nach einem der Ansprüche 2 bis 5, bei dem der Sensor (54) elektrisch mit der Verarbeitungsvorrichtung (22) verbunden und konfiguriert ist zum Erzeugen eines elektrischen Signals (50), das die Daten aufweist, die die Information bezüglich einer befohlenen Bewegung der medizinischen Vorrichtung (16) darstellen, und die Verarbeitungsvorrichtung (22) konfiguriert ist zum Empfangen des elektrischen Signals von dem Sensor.

**7.** System nach einem der Ansprüche 2 bis 6, bei dem der Sensor (54) einen optischen Sensor oder einen Magnetsensor aufweist.

**8.** System nach einem der Ansprüche 2 bis 7, bei dem die medizinische Vorrichtung (16) einen Schaft (30) aufweist, auf dem mindestens ein Marker (56) vorgesehen ist, wobei der Sensor (54) konfiguriert ist zum Detektieren einer Bewegung des mindestens einen Markers (56) und zum Erzeugen eines elektrischen Signals (50) in Antwort darauf.

**9.** System nach einem der Ansprüche 1 bis 8, bei dem die medizinische Vorrichtung (16) eine Umhüllung aufweist.

**Revendications**

**1.** Système d'initialisation ou de lancement automatique d'un algorithme de compensation de mouvement pour compenser des artéfacts de respiration, d'activité cardiaque et/ou d'autres mouvements cardiaques, comprenant
un dispositif médical (16) adapté pour un déplacement commandé incluant un cathéter comprenant un ou plusieurs capteurs de positionnement (18) pour déterminer des coordonnées brutes de localisation non compensées du cathéter ;
un appareil de traitement (22) configuré pour :

acquérir des données représentant des informations concernant un déplacement commandé du cathéter disposé au sein d'une structure anatomique ;
déterminer si ledit cathéter est dans une position anatomiquement stable d'après lesdites informations représentées par lesdites données acquises ; et
lancer automatiquement l'exécution de l'algorithme de compensation de mouvement en réponse à une détermination que ledit cathéter est dans une position anatomiquement stable pour corriger les coordonnées brutes de localisation non compensées.

**2.** Système selon la revendication 1, dans lequel ledit appareil de traitement (22) est configuré pour acquérir lesdites données provenant d'au moins l'un parmi :

un système de guidage de dispositif médical automatisé (64, 76) ;
un capteur (54) configuré pour détecter un déplacement dudit dispositif médical (16) ; et
un système de visualisation, de navigation, et/ou de cartographie (20).

**3.** Système selon la revendication 1 ou 2, dans lequel ledit appareil de traitement (22) est configuré pour déterminer si ledit dispositif médical (16) est dans une position anatomiquement stable en déterminant s'il y a eu un déplacement commandé dudit dispositif médical (16) dans une période prédéterminée de temps écoulé.

**4.** Système selon l'une quelconque des revendications 1 à 3, dans lequel ledit appareil de traitement (22) est en outre configuré pour évaluer des critères prédéterminés pour déterminer le moment où il faut lancer l'exécution de l'algorithme de compensation de mouvement, et pour lancer l'exécution de l'algorithme lorsque ledit appareil de traitement (22) détermine que ledit dispositif médical (16) est dans une position anatomiquement stable et que certains desdits critères prédéterminés sont satisfaits.

**5.** Système selon la revendication 4, dans lequel ledit appareil de traitement est configuré pour lancer l'exécution de l'algorithme de compensation de mouvement uniquement lorsqu'en plus au moins l'un des éléments suivants desdits critères prédéterminés est satisfait :

lorsqu'une distance parcourue par le dispositif médical depuis une exécution la plus récente de l'algorithme dépasse un seuil de distance prédéterminé ;
lorsqu'une durée prédéterminée s'est écoulée depuis une exécution la plus récente de l'algorithme ;
lorsqu'un déplacement commandé dudit dispositif médical (16) aboutit à une distance parcourue par ledit dispositif médical dans ladite période prédéterminée de temps écoulé qui dépasse un seuil de distance prédéterminé ;
lorsque ledit dispositif médical (16) est déterminé comme étant disposé au sein d'une région volumétrique d'une structure anatomique pour laquelle l'algorithme n'a pas été exécuté précédemment ; et
lorsque ledit appareil de traitement (22) détermine qu'un artéfact de respiration existe dans des données de localisation correspondant à une localisation dudit dispositif médical, et que ledit artéfact de respiration a une grandeur telle qu'une compensation est requise.

**6.** Système selon l'une quelconque des revendications 2 à 5, dans lequel le capteur (54) est connecté électriquement audit appareil de traitement (22) et configuré pour générer un signal électrique (50) contenant lesdites données représentant des informations concernant un déplacement commandé dudit dispositif médical (16), et ledit appareil de traitement (22) est configuré pour recevoir ledit signal électrique (50) en provenance dudit capteur.

**7.** Système selon l'une quelconque des revendications 2 à 6, dans lequel ledit capteur (54) comprend l'un d'un capteur optique et d'un capteur magnétique.

**8.** Système selon l'une quelconque des revendications 2 à 7, dans lequel ledit dispositif médical (16) comprend une tige (30) sur laquelle est disposé au moins un marqueur (56), ledit capteur (54) étant configuré pour détecter un déplacement dudit au moins un marqueur (56) et pour générer ledit signal électrique (50) en réponse à celui-ci.

**9.** Système selon l'une quelconque des revendications 1 à 8, dans lequel ledit dispositif médical (16) comprend une gaine.

FIG. 1

FIG.2

FIG.3A

FIG.3B

FIG.3C

FIG.3D

ACQUIRE DATA REPRESENTING INFORMATION RELATING TO MOVEMENT OF MEDICAL DEVICE
100

IS MEDICAL DEVICE STATIONARY ?
102
NO
YES

INITIALIZE/INITIATE EXECUTION OF ALGORITHM
104

ARE PREDETERMINED CRITERIA MET?
106
NO
YES

SET/UPDATE PARAMETERS OF ALGORITHM
108

COMPUTE COMPENSATION SIGNAL
110

APPLY COMPENSATION SIGNAL TO RAW ELECTRODE LOCATION DATA
112

FIG.4

FIG.5A

FIG.5B

FIG.6

FIG.7

FIG.8

FIG.9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040254437 A1
- US 20110160570 A1
- US 20100063514 A1
- WO 0054689 A
- US 20090037130 A1
- US 20090247993 A
- US 7263397 B
- US 6498944 B
- US 6788967 B
- US 6690963 B
- US 6233476 B
- US 7197354 B
- US 7386339 B
- US 93306310 A
- US 75184310 A
- US 34784208 A
- US 34782608 A
- US 34781108 A
- US 34744208 A
- US 2009038597 W
- WO 2009120982 A
- US 6507751 B
- US 20070016006 A1